# EUROPEAN PATENT APPLICATION

(11) **EP 4 309 678 A1**
(43) Date of publication of application: **24.01.2024**
(21) Application number: 22185972.1
(22) Date of filing: 20.07.2022
(51) Int. Cl.: A61K 49/18, A61K 41/00, A61P 35/00, A61K 35/17, A61K 39/00

(54) **LOADING OF HUMAN CAR T-CELLS WITH SUPERPARAMAGNETIC IRON-BASED PARTICLES FOR MAGNETIC TARGETING**

(71) Applicant: Friedrich-Alexander-Universität Erlangen-Nürnberg, 91054 Erlangen (DE)
(72) Inventor: ALEXIOU, Christoph, 91054 Erlangen (DE); JANKO, Christina, 91052 Erlangen (DE); DÖRRIE, Jan, 91052 Erlangen (DE); SCHAFT, Nils, 91052 Erlangen (DE)
(74) Representative: Wichmann, Hendrik

(57) **Abstract**

The application describes a T-cell expressing a chimeric antigen receptor ('CAR T-cell') containing superparamagnetic iron-based particles (loaded CAR T-cell') for use in treating a tumor. Said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions. Furthermore, an *in vitro* method of generating a CAR T-cell containing superparamagnetic iron-based particles is described, whereby such loaded CAR T-cells are generated.

## Description

### Background of the invention

Cancer represents an enormous burden to the society. Surgery, chemotherapy and radiation have represented the standard treatments. Beside checkpoint inhibitors, T-cells carrying chimeric antigen receptors (CAR) represent promising tools in cancer immunotherapy. For adoptive CAR T-cell therapy, T-cells are genetically engineered to produce CARs to recognize and kill tumor cells. Autologous T-cells are extracted from the patient by leukapheresis, ex *vivo* genetically engineered, expanded and reinfused into the patient. Especially for the treatment of hematological malignancies, CAR T-cells have already shown their great potential. In CAR T-cell therapy, the immune system effectively induces immune responses against tumors. The CAR is composed of a tumor antigen binding domain and one or more signal transduction domains, enhancing T cell proliferation and differentiation. [1] Antigen recognition by CAR T-cells is independent from MHC, meaning that immune response may get out of control and cause serious complications. [2] Thus, CAR T-cell therapy is often accompanied by serious adverse reactions such as the release of cytokines, neurotoxicity, tumor lysis syndrome, as well as off-tumor target toxicity, which can become life threatening. Therefore, research in CAR T-cells focuses on minimizing the toxicity of therapy. [3] Since nonpathogenic tissues can share antigens targeted by CAR T-cells on tumor cells, on-target/off-tumor toxicity can occur, ranging from manageable lineage depletion (B cell aplasia) to death. [4]

The therapeutic success of CAR T-cells towards solid tumors, however, has been limited due to various difficulties such as extravasation out of the blood vessel, ineffective trafficking of CAR T-cells to tumor sites, or inactivation/exhaustion of CAR T-cells by the immune suppressive tumor micro-environment. [5] Thus, new approaches are needed to improve CAR T cell tumor targeting and to reduce side effects related to unspecific targeting and/or over-activation of the immune system.

For example, melanoma represents a superficial, magnetically easy to reach target structure for T cell accumulation. Chondroitin sulfate proteoglycan 4 (CSPG4) represents a cell-surface antigen not only expressed on 90% of melanoma primary tumors and metastases, but also on other tumors such as sarcomas, astrocytomas, gliomas, and neuroblastomas and leukemia. [6-10]

Despite the expression level of CSPG4 is up to 100-fold higher on melanoma cells, CSPG4 is also expressed on healthy cells such as chondrocytes of the articular cartilage, smooth muscle cells, cells of the neuromuscular synapse of postnatal skeleton muscles and fetal melanocytes. [11-13]

Thus, unspecific systemic distribution is prone to cause severe side effects and targeting of the CAR T-cells to the tumor region might decrease toxicity and increase efficacy. The inventors have previously generated a CAR specific for CSPG4. T-cells transfected with RNA encoding a CAR specific for CSPG4 have proofed effective in antigen-specific tumor cell lysis and release of pro-inflammatory cytokines. [14] However, said CAR T-cells have not been targeted to the site of the tumor.

Superparamagnetic iron-based particles have been described in the art and can serve as contrast agents for magnet resonance imaging (MRI), magnetically guidable drug transporters or as tool to make cells targetable by magnetic forces. Magnetized cells have previously been used for the magnetic delivery of endothelial cells, stem cells, or dendritic cells. [15-17] In case of lymphocytes, magnetic labelling has been performed to follow their migration by MRI. The inventors have previously functionalized lymphocytes with superparamagnetic iron-based particles to enable their magnetic control. [18-21]

Hence there is a need in the art to target CAR T-cells to the site of the tumor to reduce off-target effects of the CAR T-cells. Using dynamically programmable electromagnetic fields, Pai et al. were able to non-invasively aggregate therapeutic cells such as neural stem cells, monocytes or CAR T-cells at a particular site. [22] The inventors have previously loaded primary human T-cells with superparamagnetic iron oxide nanoparticles (SPION) and SPION loading of T-cells did not impair their effector functions after polyclonal stimulation as shown previously. [18, 21] CAR-T cells expressing the CAR permanently (generated by viral transduction) have been loaded with Ferumoxytol for tracking tumor infiltration. There it has been shown that these Ferumoxytol-loaded CAR-T cells preserved proliferation, viability and function (such as release of IFNγ). [23]

Based on Mühlberger et al. [21] , it is unclear whether CAR T-cells would maintain their tumor killing capacity, if loaded with superparamagnetic iron-based particles. Furthermore, it is unclear whether loaded CAR T-cells would show increased, equal or decreased levels of cytokine production and release compared to unloaded CAR T-cells. As T-cells did not show an impairment in effector functions, the skilled person might have considered that CAR T-cells may have an equal cytokine production and release.

### Summary of invention

The present invention is directed to a T-cell expressing a chimeric antigen receptor ('CAR T-cell') containing superparamagnetic iron-based particles ('loaded CAR T-cell') for use in treating a tumor, wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions, as defined in the claims.

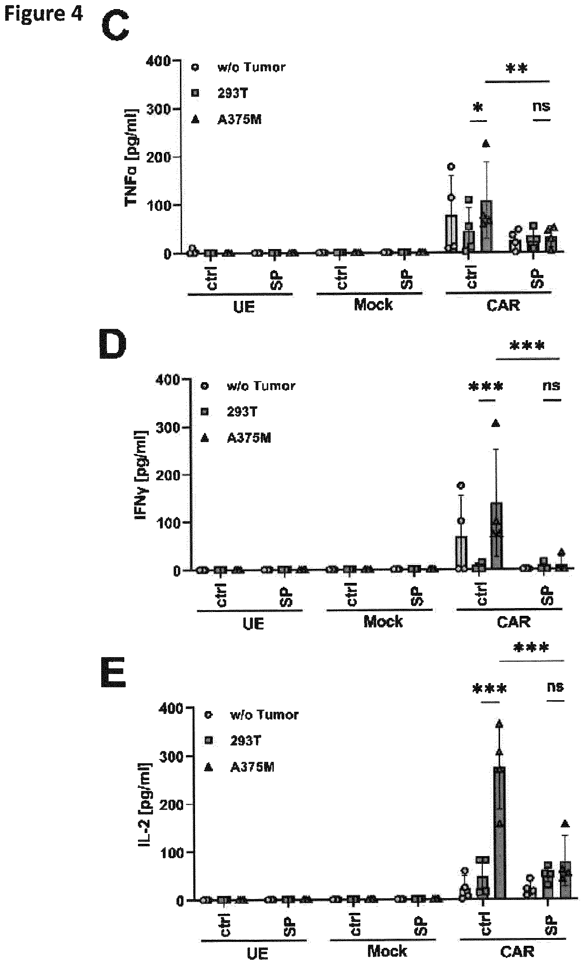

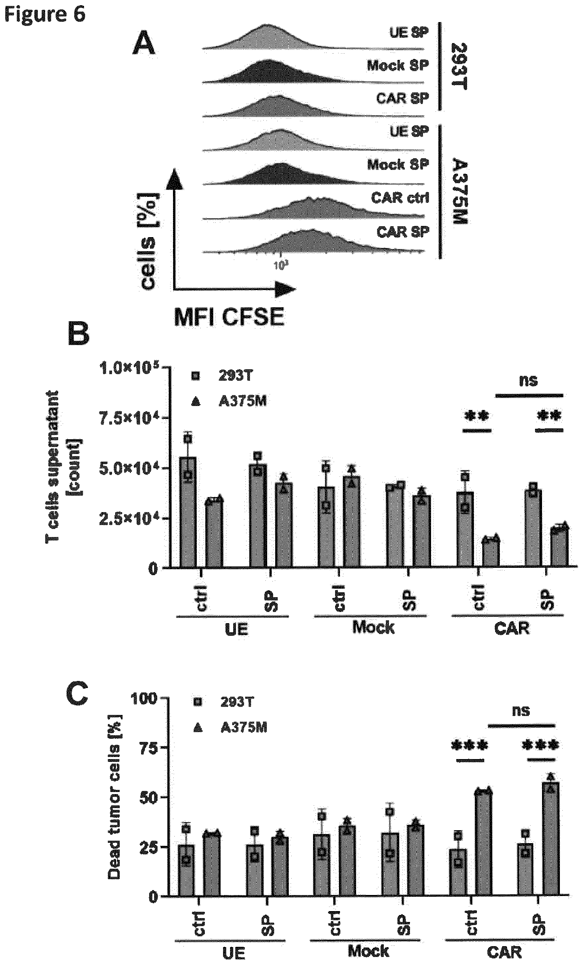

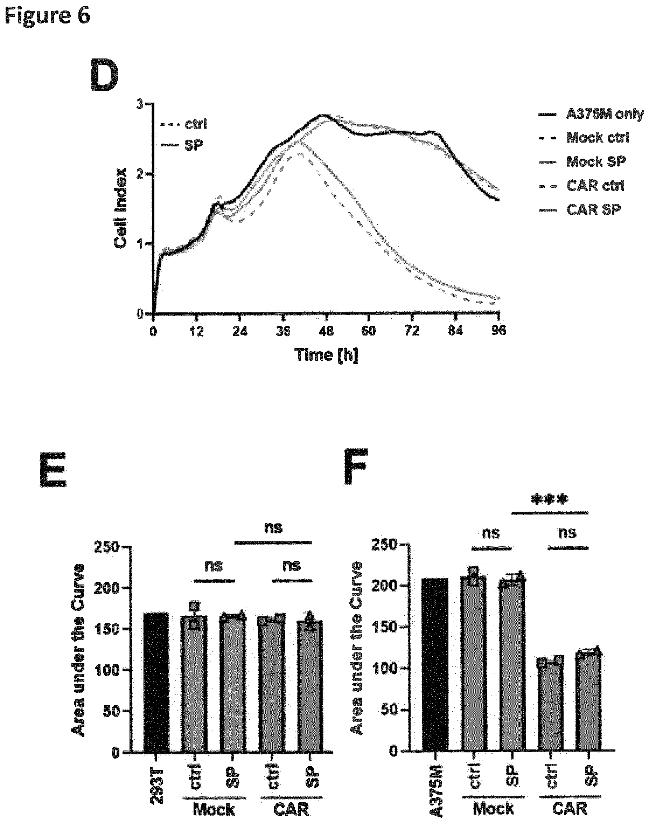

Loaded CAR T-cells are magnetically attractable/movable, while maintaining their tumor killing capacity. In particular Fig. 6 herein demonstrates that the tumor killing capacity of these CAR T-cells is maintained. As an example, tumor cells and multicellular tumor spheroids are lysed by the SPION-loaded CAR T-cells with the same efficiency as by unloaded CAR T-cells, which is demonstrated in **Fig. 6****.** Interestingly, these loaded CAR T-cells (CAR T-cells containing superparamagnetic iron-based particles) exhibit a reduced cytokine release. In particular, Fig. 4C-E exemplify that the cytokine release of CAR T-cells containing superparamagnetic iron-based particles is significantly reduced. Loaded CAR T-cells surprisingly inhibited antigen-specific release of inflammatory cytokines (e.g. TNFalpha, IFNgamma, IL-2), as demonstrated in Fig. 4C-E as an example.

Since a massive systemic release of cytokines, termed cytokine release syndrome or cytokine storm, is the most frequent dangerous side effects of CAR T-cell therapy [24], it would be medically advantageous to use CAR T-cells which display a reduced cytokine release activity, while still maintaining their other effector functions. Reducing cytokine release is expected to reduce the systemic inflammatory side effects of CAR T-cell therapy, most importantly the dreaded cytokine release syndrome. The reduction of side effects as well as the reduction of the risk of a cytokine storm/cytokine release syndrome is crucial in CAR T-cell therapy, as such side effects can be systemic and in some cases even fatal. [24] Hence, the reduced antigen-specific cytokine release of the CAR T-cells, containing superparamagnetic iron-based particles, is expected to reduce systemic side effects caused by excessive inflammatory immune reactions.

In sum, the inventors demonstrated that loaded CAR T-cells retain the capacity (i) to locally kill tumor cells while (ii) reducing the release of inflammatory cytokines. Furthermore, the cells acquire (iii) magnetic movability so that they can be navigated to the tumor site using a magnetic field.

The present disclosure is also directed to a CAR T-cell containing superparamagnetic iron-based particles (a 'loaded CAR T-cell'), wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions, as defined in the claims.

The present disclosure is also concerned with an *in vitro* method of generating a CAR T-cell containing superparamagnetic iron-based particles (a 'loaded CAR T-cell') comprising, preferably consisting of, the steps
a. introducing a CAR mRNA into a T-cell; and
b. loading the T-cell of step (a) with superparamagnetic iron-based particles whereby the loaded CAR T-cell is generated.

### Detailed description of the Invention

The present disclosure is concerned with a CAR T-cell containing superparamagnetic iron-based particles (a 'loaded CAR T-cell') for use in treating a tumor, wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions, as defined in the claims. In other words, a CAR T-cell containing superparamagnetic iron-based particles is a loaded CAR T-cell. A CAR T-cell not containing superparamagnetic iron-based particles is an unloaded CAR T-cell.

A CAR T-cell for use in treating a tumor is also disclosed, wherein said CAR T-cell has been loaded with superparamagnetic iron-based particles, wherein said CAR T-cell exhibits a reduced cytokine release upon binding to a cell of the tumor by the CAR T-cell compared to CAR T-cell which is not loaded with superparamagnetic iron-based particles under the same conditions.

As noted above, the present invention is concerned with the particular advantage that loaded CAR T-cells exhibit less cytokine release upon binding to the cell of the tumor and thus the risk of excessive and harmful cytokine release is reduced. Especially, the risk of a potentially fatal cytokine storm/cytokine release syndrome is reduced by the invention. This is a new finding of the inventors, as it has not been known or anticipated that CAR T-cells loaded with superparamagnetic iron-based particles exhibits such a reduction in the cytokine release. The reduced cytokine release is corroborated especially by Fig. 4C-E showing that the cytokine release of loaded CAR T-cells is reduced compared to unloaded CAR T-cells. It is thus expected to be safer for patients to use loaded CAR T-cells as the risk of a cytokine storm/ cytokine release syndrome is reduced. Furthermore, it is also expected that the patients suffer from less side effects such as inflammation when treated with loaded CAR T-cells compared to unloaded CAR T-cells. This is because the CAR T-cells release less cytokines and thus less general inflammation in the patient is elicited. Hence, it is also expected that the patient is suffering from less unpleasant side effects. Furthermore, as the loaded CAR T-cells exhibit a reduced cytokine release and thus the inflammation is reduced, it is also expected that the dose of CAR T-cells in a patient can be increased compared to unloaded CAR T-cells. Furthermore, as the loaded CAR T-cells can be monitored, e.g. via MRI, the dose of CAR T-cells can also be adjusted on an individual basis. For example, the clearance rate depends on the patient. As the loaded CAR T-cells can be monitored by MRI, the amount of CAR T-cells at the tumor site and at other sites in the body can be monitored during application of the CAR T-cells, during the focusing to the tumor site, and during the tumor killing action of the CAR T-cells. In case of a patient with a high clearance rate, further CAR T-cells can be injected in order to ensure a suitable amount of CAR T-cells at the tumor site.

At the same time, loaded CAR T-cells are magnetically attractable and can thus be directed to the site of the tumor in a patient. In contrast thereto, unloaded CAR T-cells are not magnetically attractable and can thus not be directed to the site of the tumor with a magnet. The magnetic attractability allows a targeted concentration of the CAR T-cells at the site of the tumor. This is particularly advantageous as CAR T-cells often elicit immune reactions at non-tumorous sites of the body, which poses additional stress to the human body. Thus, the magnetic attractability is a further advantage of the present invention.

Generally, the skilled person is aware that 'cytokines' are a broad category of oligo- and polypeptides important in cell signaling. Specifically, cytokines are peptides, which cannot cross the lipid bilayer of cells to enter the cytoplasm. Cytokines are involved in autocrine, paracrine and endocrine signaling as immunomodulating agents. For example, chemokines, interferons, interleukins, lymphokines, and tumor necrosis factors are types of cytokines.

According to the present disclosure, the cytokine release may comprise the release of TNFalpha, IFNgamma and/or IL-2. Fig. 4C-E of the present disclosure provides data on the release of TNFalpha, IFNgamma and/or IL-2. In a preferred embodiment, the cytokine release comprises at least two of TNFalpha, IFNgamma and/or IL-2. It is even more preferred that the cytokine release comprises TNFalpha, IFNgamma and IL-2.

As defined in the claims, the cytokine release is reduced in CAR T-cells containing superparamagnetic iron-based particles compared to CAR T-cells not containing superparamagnetic iron-based particles. In particular, the reduced cytokine release may be at least reduced by 20% and preferably at least 30%. It is even more preferred that the cytokine release is reduced by at least 40%, and even more preferably at least 50%, compared to cytokine release by the unloaded CAR T-cell.

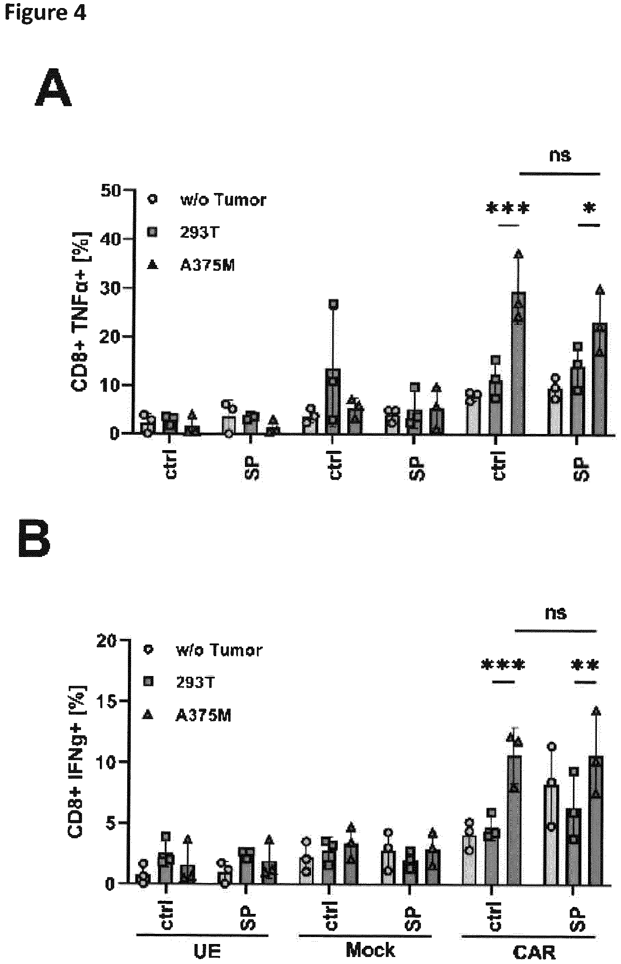

The skilled person is aware of methods to measure cytokine release. For example, the skilled person may measure the cytokine release in an *in vitro* assay. Said assay may be an enzyme-linked immunosorbent assay (ELISA), an enzyme-linked immune absorbent spot (ELISpot), Fluorospot assay and a bead based immunoassay. The skilled person is aware of exemplary bead based immunoassays, which are commercially available, for example from Legendplex. As also shown in Example 1 and especially Fig. 4, a bead based immunoassay is preferred to measure cytokine release. Alternatively, an ELISA may be used to measure cytokine release.

ELISA and bead-based immunoassays are also applicable for determination of cytokine release from blood samples of treated individuals.

The reduced cytokine release may be measurable by an assay performed on patient blood samples. An *in vitro* assay performed on patient blood samples may be selected from an enzyme-linked immunosorbent assay (ELISA), and a bead based immunoassay. Specifically, patient serum or plasma may be assessed. It is strongly preferred that an in vitro ELISA assay is used.

Such measurements may be accompanied by measuring the patient's vital parameters. For example, the reduced cytokine release may further be measurable by monitoring one or more vital parameters. The skilled person is aware of suitable vital parameter. Exemplary parameters to monitor cytokine release are patient's body temperature and/or C-reactive protein (CRP) level. For example, the patient's body temperature may be below 38 °C. It is also preferred that the patient's CRP level is below 10 mg/l. Standard methods to measure body temperature and CRP level are known in the art. Ideally, the patient's body temperature remains below 38°C and/or the CRP level remains below 10 mg/l. It is preferred that the patient's body temperature remains below 38°C and the CRP level remains below 10 mg/l.

In particular, the reduced cytokine release may comprise a reduced TNFalpha release. The skilled person is aware of standard methods such as an ELISA to measure TNFalpha release. Furthermore, commercial antibodies against TNFalpha are available, which the skilled person is aware of. For further guidance, Example 1, Fig. 4 provides exemplary experimental details and supporting data. It is preferred that the reduced TNFalpha release is at least reduced by 20%, preferably at least 30%, more preferably at least 40%, more preferably at least 50%, and most preferably at least 60%, compared to cytokine release by the unloaded CAR T-cell. Said comparison may be carried out under otherwise same conditions.

Similarly, the reduced cytokine release may comprise a reduced IFNgamma release. The skilled person is aware of standard methods such as an ELISA to measure IFNgamma release. Furthermore, commercial antibodies against IFNgamma are available, which the skilled person is aware of. For further guidance, Example 1, Fig. 4 provides exemplary experimental details and supporting data. It is preferred that the reduced IFNgamma release is at least reduced by 20%, preferably at least 30%, more preferably at least 40%, more preferably at least 50%, more preferably at least 60%, even more preferably at least 70%, and most preferably at least 80%, and most preferably at least 90%, compared to cytokine release by the unloaded CAR T-cell. Said comparison may be carried out under otherwise same conditions.

Furthermore, the reduced cytokine release may comprise a reduced IL-2 release. The skilled person is aware of standard methods such as an ELISA to measure IL-2 release. Furthermore, commercial antibodies against IL-2 are available, which the skilled person is aware of. For further guidance, Example 1, Fig. 4 provides exemplary experimental details and supporting data. It is preferred that the reduced IL-2 release is at least reduced by 20%, preferably at least 30%, more preferably at least 40%, more preferably at least 50%, even more preferably at least 60%, and most preferably at least 70%, compared to cytokine release by the unloaded CAR T-cell. Said comparison may be carried out under otherwise same conditions.

It is a particular advantage of the present disclosure that the reduced cytokine release results in a reduced risk of an excessive inflammatory immune reaction. In some cases, said excessive inflammatory immune reaction may be a cytokine storm, which may be fatal to the patient. Hence, it is preferred that the reduced cytokine release results in a reduced risk of an excessive inflammatory immune reaction, wherein the excessive inflammatory immune reaction may be a cytokine storm.

Specifically, CAR-T-cells usually induce pyroptosis, an inflammatory form of cell death in the target cells. Pyroptosis is characterized by release of damage-associated molecular patterns (DAMPs: ATP, HSPs, HMGB1) from dying cells. Pyroptosing tumor cells trigger macrophages to greatly release pro-inflammatory cytokines (such as IL-1β, IL-6), causing the cytokine release syndrome (CRS). As shown in Fig. 4 herein, the loaded CAR T-cells exhibit a reduced cytokine release, the pyroptosis by tumor cells is expected to be reduced. In one embodiment, the reduced cytokine release reduces pyroptosis of the tumor cells.

As cytokine and/or DAMP release by dying tumor cells can elicit a cytokine storm caused by activated macrophages, a reduced cytokine release by the CAR T-cells is expected to have an advantageous impact on therapeutic use of loaded CAR T-cells.

Preferably, the superparamagnetic iron-based particles inhibit the cytokine release from the loaded CAR T-cell. As shown in Fig. 4 C-E, the cytokine release is reduced in loaded CAR T-cells compared to unloaded CAR T-cells upon binding to tumor cells under the same conditions.

In general, the term CAR T-cell is a 'chimeric antigen receptor T cell, which is a T cell that has been genetically engineered to produce an artificial T cell receptor for use in immunotherapy. The skilled person is aware of CAR T-cells. Chimeric antigen receptors (CARs, also known as chimeric immunoreceptors, chimeric T cell receptors or artificial T cell receptors) are receptor proteins that have been engineered to give T-cells the new ability to target a specific protein. The receptors are chimeric because they combine both antigen-binding and T cell activating functions into a single receptor.

CAR T-cells are used for cancer therapy. Generally, the premise of CAR T immunotherapy is to modify T-cells to recognize cancer cells in order to more effectively target and destroy them. In very general terms, scientists harvest T-cells from patients, genetically alter said cells, then infuse the resulting CAR T-cells into patients to attack their tumors.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (wherein the latter is referred to herein as "an intracellular signaling domain") comprising a functional signaling domain derived from a stimulatory molecule and/or costimulatory molecule as defined below. In some embodiments, the set of polypeptides are contiguous with each other. In some embodiments, the set of polypeptides include a dimerization switch that, upon the presence of a dimerization molecule, can couple the polypeptides to one another, e.g., can couple an antigen binding domain to an intracellular signaling domain. It is preferred that the stimulatory molecule is the zeta chain (ζ) associated with the T cell receptor complex. In particular, the cytoplasmic signaling domain may further comprise one or more functional signaling domains derived from at least one co-stimulatory molecule. For example, the co-stimulatory molecule is chosen from the costimulatory molecules described herein, e.g., 4-1BB (i.e., CD137), CD27 and/or CD28. The CAR may comprise a chimeric fusion protein comprising an extracellular antigen binding domain, a transmembrane domain and an intracellular signaling domain comprising a functional signaling domain derived from a stimulatory molecule.

The skilled person is also aware that there are different types, also called generations of CAR T-cells. Specifically, the first generation of CARs usually contained a single CD3ζ intracellular domain. It is known that initial experiments with first-generation CAR T-cells showed low cytotoxicity and proliferation owing to the lack of co-stimulatory (e.g. CD27, CD28, CD134, CD137) and cytokine (e.g. interleukin (IL)-2) signalling. A second generation of CARs was generated to enhance T cell proliferation and cytotoxicity by adding a co-stimulatory domain such as sections of CD28 or CD137 to the intracellular signalling domain. The third generation of CARs, which is also known to the skilled person, further expanded on the second generation by adding a third intracellular signalling sequence of an additional co-stimulatory domain such as CD134 or CD137. The fourth generation of CARs is based on second-generation CARs, but includes a cytokine, such as interleukin 12 (IL-12) that is constitutively or inducibly expressed upon CAR activation. T-cells transduced with these fourth-generation CARs are referred to as T-cells redirected for universal cytokine-mediated killing (TRUCKs). Activation of these CARs promotes the production and secretion of the desired cytokine to promote tumor killing though several synergistic mechanisms such as exocytosis (e.g. perforin, granzyme) or death ligand-death receptor (e.g. Fas-FasL) systems. A fifth generation is based on the second generation of CARs, but they contain a truncated cytoplasmic IL-2 receptor β-chain domain with a binding site for the transcription factor STAT3. Additional variants of the aforementioned CARs, such as dual CARs, split CARs and inducible-split CARs, have been generated to further enhance the specificity and control of the transfused T-cells.

The CAR T-cell according to the present disclosure may be a first, second, third, fourth or fifth generation CAR T-cell. It is preferred that the CAR T cell according to the present disclosure is a second generation CAR T-cell. It is even more preferred that said second generation CAR T-cell comprises a co-stimulatory domain and an intracellular signaling domain, wherein the co-stimulatory domain comprises sections of CD28 or 4-1BB. In an especially preferred embodiment, the co-stimulatory domain comprises section of CD28. In particular, the co-stimulatory domain may comprise a section of CD28 and the intracellular signaling domain may comprise a section of CD3ζ, thereby forming a CD28-CD3ζ CAR.

In general, "superparamagnetic iron-based particles" as used herein are particles, which are magnetically attractable. The skilled person is aware that the magnetic behaviour of materials can be classified into five major groups: ferro-, ferri-, para-, antiferro-, and diamagnetism. Ferro- and ferrimagnetism are the basic mechanisms in permanent magnets. Permanent magnets can be magnetized by external magnetic fields and remain magnetized after the removal of the fields. Materials such as Fe (iron), Co, Ni, and their alloys, and some compounds of rare earth elements are ferromagnetic. Superparamagnetism is a form of magnetism, which appears in small ferromagnetic or ferrimagnetic particles. "Particles" as used herein are defined as particles of matter that are from 1 nm (nanometer) to 2 micrometers (µm) in diameter. Furthermore, the skilled person is aware of iron (Fe) based particles, such as iron oxide based particles, which have been used in biological applications. The skilled person is also aware of various iron-based particles. For example, the particles may be iron oxide, iron (Fe), iron-cobalt, alnico or permalloy particles. In other words, the superparamagnetic iron-based particles may be selected from iron oxide, iron (Fe), iron cobalt, alnico, and permalloy particles. In a preferred embodiment, the superparamagnetic iron-based particles are superparamagnetic iron oxide nanoparticles (SPIONs). A "nanoparticle", as used herein, is a particle of matter that is between 1 nm and 1 µm in diameter. Furthermore, the superparamagnetic iron-based particles may be superparamagnetic iron oxide nanoparticles (SPIONs), more preferably wherein the iron oxide is selected from Fe₃O₄, γ-Fe₂O₃, CoFe₂O₄ or a mixture thereof, more preferably wherein the iron oxide is Fe₃O₄. In a preferred embodiment, the concentration of the superparamagnetic iron-based particles is determined by atomic emission spectroscopy (AES). The iron content in a solution may, for example, be determined with Atomic Emission Spectroscopy (AES). The skilled person is also aware of other methods in order to determine the iron content in a sample. For example, the concentration of iron in a sample can also be determined photometrically. However, a determination of the iron concentration by AES is preferred.

In a preferred embodiment, the superparamagnetic iron-based particles have been coated with citrate, oxalic acid, ascorbic acid, fatty acids and/or amino acids before use according this disclosure. A coating with citrate is especially preferred. For example, the superparamagnetic iron-based particles may have been coated with citrate following the protocol as described in Mühlberger et al. [19]

Superparamagnetic iron-based particles may have been coated with citrate, as shown in **Example 1** herein. Citrate coated superparamagnetic iron-based particles have the advantage that they can easily be purified. Furthermore, the purification method has been advanced in contrast to Mühlberger et al., which was washed with acetone five times and then dried under vacuum. [21] Specifically, the superparamagnetic iron-based particles herein have been cleaned of excess molecules by aqueous washing steps based on porous membrane filtration followed by an ultrasound treatment. The advantage of aqueous washing steps based on porous membrane filtration is that said method is more easily up-scalable for later clinical use and said method does not have the risk of acetone impurities, which would hamper the clinical use. It is thus preferred that the superparamagnetic iron-based particles have been cleaned by aqueous washing and filtration through porous membranes before use.

With regard to exemplary preparation of the superparamagnetic iron-based particles, reference is made to Example 1. Preferably, the superparamagnetic iron-based particles have been cleaned of excess molecules by aqueous washing based on porous membrane filtration followed by an ultrasound treatment before use. The exemplary superparamagnetic iron-based particles of Example 1 have also been purified in this way. The skilled person can determine suitable ultrasound treatment parameters. It is preferred that the ultrasound treatment has been performed with at least 1.25 kJ/mL and at most 10 kJ/mL of energy density.

After preparation, the superparamagnetic iron-based particles are ready for loading CAR T-cells. Ideally, the superparamagnetic iron-based particles have a hydrodynamic diameter of at most 120 nm. It is preferred that the hydrodynamic diameter is at most 100 nm, more preferably at most 90 nm, more preferably at most 80 nm, and more preferably at most 75 nm. A suitable minimal hydrodynamic diameter might be at least 35 nm.

The superparamagnetic iron-based particles may also be detectable by MRI. Detection by MRI has the particular advantage that the superparamagnetic iron-based particles can be followed in the patient and the magnetic attractability to the tumour may be monitored. The detection by MRI has therefore diagnostic value. In addition, the magnetic field of the MRI may be used to further focus the CAR T-cells towards the tumor via the magnetic field. Hence, the MRI may also have therapeutic value. Preferably, the loaded CAR T-cell is a theranostic agent. The skilled person is aware that theranostics are generally a combination of the term therapeutics and diagnostics. It is more preferred that said theranostic agent is visible by MRI or ultrasound. Ideally, the MRI or ultrasound allows imaging controlled therapy. This means that the loaded CAR T-cells can be followed by the clinician in a patient. This has the advantage that the loaded CAR T-cells may be localized in the patient's body, such as at the site of the melanoma. It is even more preferred that said theranostic agent is visible by MRI. In this case, the MRI may allow imaging guided therapy. This means that the loaded CAR T-cells can be focused by the clinician in a patient. This has the advantage that the loaded CAR T-cells may be specifically guided in the patient's body, such as to the site of the melanoma.

In a preferred embodiment, the superparamagnetic iron-based particles are detected by MRI. One key advantage of loaded CAR T-cells is that they are detectable in MRI as superparamagnetic iron-based particles are negative contrast agents. Hence, superparamagnetic iron-based particles lead to a dark signal in MRI. This offers the advantages of magnetic targeting of the loaded CAR T-cells to the desired position in the patient. Furthermore, the therapy success can be closely monitored as the presence and absence of a suitable amount of loaded CAR T-cells at the tumor site can be monitored. Thus, the loaded CAR T-cells serve as theranostic agents. The detectability of superparamagnetic iron-based particles depends on their transversal (T2 and T2^{∗}) relaxivity. In general, when detecting loaded CAR T-cells using MRI, the T2^{∗} relaxation time of loaded CAR T-cells may be reduced compared to the unloaded CAR T-cell under the same conditions.

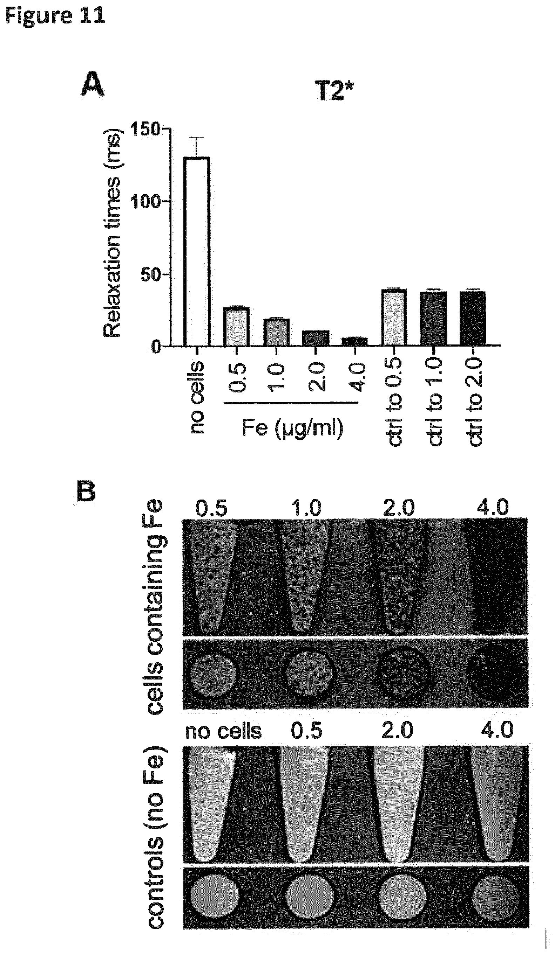

In general, the skilled person is aware that T2^{∗}-weighted imaging is an MRI sequence to quantify observable or effective T2 (referred to as T2^{∗} or "T2-star"). T2^{∗} can be considered an "observed" or "effective" T2, whereas T2 may be considered the "natural" or "true" T2 of the tissue being imaged. T2^{∗} is always less than or equal to T2. It is known that T2^{∗} results principally from inhomogeneities in the main magnetic field. These inhomogeneities may be the result of intrinsic defects in the magnet itself or from susceptibility-induced field distortions produced by the tissue or other materials placed within the field. Certain MR imaging sequences using gradient echoes, which are called T2^{∗}-weighted. These sequences are used to accentuate local magnetic homogeneity effects to aid in the detection of e.g. hemorrhage or calcifications, but also superparamagnetic iron-based particles according to the present disclosure. Particular reference is made to **Example 2** and **Fig. 11****,** wherein loaded CAR T-cells were visualised in MRI. Furthermore, in Example 3, point 2.2 herein, the MRI imaging of the loaded CAR T-cells in a patient is described.

It is especially preferred that the T2^{∗} relaxation time is reduced by at least 30 % compared to the unloaded CAR T-cell under the same conditions, even more preferably 40% compared to the unloaded CAR T-cell under the same conditions, and most preferably at least 50% compared to the unloaded CAR T-cell under the same conditions.

In general, CAR T-cells do not contain superparamagnetic iron-based particles. In order to introduce superparamagnetic iron-based particles into CAR T-cells, CAR T-cells may be incubated with superparamagnetic iron-based particles before use. The CAR T-cells engulf superparamagnetic iron-based particles by binding to the plasma membrane and/or endocytosis. Hence, CAR T-cells are capable of accumulating superparamagnetic iron-based particles. The loading can be enhanced by a preceding electroporation step. In general, electroporation facilitates the uptake of substances, such as plasmids. Similarly, lipofectamine and PEI also facilitate the uptake of substances.

The superparamagnetic iron-based particles may have been introduced into the CAR T-cell by incubating the CAR T-cell with the superparamagnetic iron-based particles. Alternatively, the superparamagnetic iron-based particles may have been introduced into the CAR T-cell by incubation of the CAR T-cells with superparamagnetic iron-based particles after electroporation, by treatment with an effective amount of lipofectamine and/or by treatment with an effective amount of PEI. Of course the skilled person is also aware of alternative methods to introduce non-endogenous objects such as superparamagnetic iron-based particles into T-cells. It is preferred that the superparamagnetic iron-based particles have been introduced by incubation of the CAR T-cells with superparamagnetic iron-based particles after electroporation. Exemplary data of such an introduction is provided in Example 2.

In general, the loaded CAR T-cell comprises a suitable amount of superparamagnetic iron-based particles. A minimal suitable amount is present when the cells are still magnetically attractable. A maximal suitable amount is present when the cells still show normal morphology and viability. An unsuitable/excessive amount is present when the cells do not show normal morphology and viability. The skilled person is aware of standard methods to measure the amount of superparamagnetic iron-based particles in cells. For example, AES can be used to assess the amount of iron (Fe). It is preferred that the loaded CAR T-cell comprises from 0.5 to 4 pg iron per cell (Fe/cell), preferably from 0.55 to 3.5 pg Fe/cell, more preferably from 0.6 to 3.3 pg Fe/cell, more preferably from 0.65 to 3.1 pg Fe/cell, more preferably from 0.7 to 2.9 pg Fe/cell, more preferably from 0.8 to 2.7 pg Fe/cell, more preferably from 0.9 to 2.6 pg Fe/cell, more preferably from 1 to 2.5 pg Fe/cell, and most preferably around 1 pg Fe/cell.

In general, after CAR T-cells are infused into a patient, said cells act as a 'living drug' against cancer cells. When they come in contact with their targeted antigen on the tumor cell, CAR T-cells bind to it and become activated, then proceed to proliferate and become cytotoxic. CAR T-cells destroy tumor cells through several mechanisms, including extensive stimulated cell proliferation, increasing the degree to which they are toxic to other living cells (cytotoxicity) and by causing the increased secretion of factors that can affect other cells such as cytokines, interleukins and growth factors. According to the present disclosure, the loaded CAR T-cell is stimulated upon binding to a cell of the tumor by the CAR T-cell. At the same time, the loaded CAR T-cell exhibits a reduced cytokine release compared to an unloaded CAR T-cell under the same conditions.

The stimulation of the loaded CAR T-cell is for example manifested by the intracellular cytokine production. In embodiments, both the loaded and the unloaded CAR T-cell exhibit cytokine production intracellularly. However, the amount of intracellular cytokine production may be slightly different. Specifically, the loaded CAR T-cell may exhibit a reduced intracellular cytokine production upon binding to a cell of the tumor by the CAR T-cell compared to the unloaded CAR T-cell under the same conditions. For example, the intracellular cytokine production of the loaded CAR T-cell may be reduced by at most 30 %, more preferably at most 25%, more preferably at most 20%, more preferably at most 15%, more preferably at most 12%, more preferably at most 10%, and even more preferably at most 8%. In particular, Fig. 4 A and B show that the intracellular cytokine production may be slightly reduced in loaded CAR T-cells compared to unloaded CAR T-cells. However, the reduction was in both measured cases insignificant. Hence, it is preferred that the loaded CAR T-cell exhibits a reduced cytokine production to an insignificant extent upon binding to a cell of the tumor by the CAR T-cell compared to the unloaded CAR T-cell under the same conditions. It is even more preferred that insignificance is assessed by a 2-way ANOVA with a posthoc Tukey test, wherein p>0.05 is insignificant.

Furthermore, the loaded CAR T-cell exhibits similar tumor killing efficacy compared to the unloaded CAR T-cell under the same conditions. **Fig. 6** demonstrates that the tumor killing efficacy showed an insignificant difference between the loaded and the unloaded CAR T-cells. Hence, it is preferred that the loaded CAR T-cell exhibits similar tumor killing efficacy upon binding to a cell of the tumor by the CAR T-cell compared to the unloaded CAR T-cell under the same conditions. It is even more preferred that the similar tumor killing efficacy is defined as an insignificant difference in tumor killing efficacy. It is even more preferred that insignificance is assessed by a 2-way ANOVA with a posthoc Tukey test, wherein p>0.05 is insignificant.

As described above, the CAR T-cell is preferably magnetically attractable. The skilled person is aware of standard methods to assess magnetic attractability. Ideally, the loaded CAR T-cell is magnetically attractable to the organ of interest. It is preferred that the loaded CAR T-cell is magnetically guidable. Specifically, the loaded CAR T-cell may be magnetically guidable to an organ of interest. In general, any organ can be an organ of interest. An organ of interest in the present case comprises a tumor. Exemplary organs of interest can be skin, liver, pancreas, spleen, kidneys, adrenal glands, stomach, brain, intestines, gallbladder, bladder, rectum, lung, heart. The tumor may be in a part of the organ. Hence, the loaded CAR T-cells can be directed to said part of the organ. In case of skin, the loaded CAR T-cells are magnetically attractable to a particular region of the skin.

As noted above the loaded CAR T cell is magnetically attractable with a magnetic field. The skilled person is aware of suitable magnetic fields, which can be used on a patient. Exemplary magnetic field strengths range from 0.01 Tesla to 7 Tesla, preferably 0.02 Tesla to 6 Tesla, more preferably 0.03 Tesla to 5 Tesla, more preferably 0.04 Tesla to 4 Tesla, more preferably 0.05 Tesla to 3 Tesla, more preferably 0.05 Tesla to 2 Tesla, more preferably 0.05 Tesla to 1.5 Tesla, more preferably 0.07 Tesla to 1.2 Tesla, more preferably 0.1 Tesla to 1.1 Tesla, more preferably 0.2 Tesla to 1.05 Tesla, more preferably 0.3 Tesla to 1 Tesla, more preferably 0.4 Tesla to 1 Tesla more preferably 0.5 Tesla to 1 Tesla, more preferably 0.6 Tesla to 1 Tesla, more preferably 0.7 Tesla to 1 Tesla, and even more preferably around 1 Tesla.

In contrast, the unloaded CAR T-cell has not been loaded with superparamagnetic iron-based particles. Hence, the unloaded CAR T-cell is not magnetically attractable. CAR T-cells may naturally contain minimal amounts of iron per cell. For example, iron-sulphur clusters and other chemical compound naturally occurring in cells contain small amounts of iron.

In particular, the unloaded CAR T-cell may comprise at most 0.1 pg Fe/cell. The skilled person is aware of suitable methods to measure the amount of iron per cell. Ideally, the amount of iron per cell is measurable by determining the cell number of CAR T-cells per volume and determining the amount of iron in a lysed sample of CAR T-cells. For example, the cellular iron concentrations (pg/cell) may be calculated using the cell count and the Fe amount in the cell lysates (ng/ml). When using, e.g. AES to measure the iron content a standard curve for calibration is usually used. For the data provided in Example 1, the lowest standard in the calibration curve is 25 ng/ml. The iron concentration in the cell lysates of loaded CAR T-cells was typically around 50 ng/ml according to Example 1.

The skilled person is aware that cells naturally contain iron, which is important for cellular functions. Further detail on this approach can be found in Example 1 herein. For example, the amount of CAR T-cells is measurable by flow cytometry and/or the amount of iron is measurable by AES.

In general, the medical use as described herein is intended to treat a patient having a tumor. A "patient" or "subject" for the purposes of the present invention is used interchangeably and meant to include both humans and other animals, particularly mammals, such as pets including cats, dogs and horses, and other organisms. Thus, the methods are applicable to both human therapy and veterinary applications. In the preferred embodiment the patient or subject is a mammal, and in the most preferred embodiment the patient or subject is a human.

The CAR T-cell therapy is ideally applied in a therapeutically effective amount. A "therapeutically effective amount" refers to that amount which provides a therapeutic effect for a given condition and administration regimen. In particular, "therapeutically effective amount" means an amount that is effective to reverse, alleviate or ameliorate symptoms of the tumor or prolong the survival of the subject being treated, which may be a human or non-human animal. Determination of a therapeutically effective amount is within the skill of the person skilled in the art. In particular, in the present case a "therapeutically effective amount" refers to the amount of CAR T-cells which, when administered to a human or animal, leads to a therapeutic effect in said human or animal. The effective amount is readily determined by one of skill in the art following routine procedures.

The loaded CAR T-cell bind to a cell of the tumor according to this disclosure. Ideally, the loaded CAR T-cell targets a solid tumor. Exemplary solid tumors melanoma, sarcoma, astrocytoma, glioma, neuroblastoma. Ideally, the tumor is located near the surface of the patient so that targeting by the magnetic field is more convenient. Thus, melanomas and conveniently-located surface-near sarcomas, gliomas, and neuroblastomas are preferred according to the present disclosure.

The loaded CAR T-cell is specific for a tumor marker. The skilled person is aware of suitable tumor markers for solid tumors. For example, the loaded CAR T-cell may be a CSPG4-specific, a HER2-specific, an EGFRvIII-specific, a PSMA-specific, a CEA-specific, a MUC1-specific, a CD19-specific, a CD20-specific, a CD22-specific, a CD30-specific, a CD269-specific, or a CD138-specific CAR T-cell. According to the present disclosure, a CSPG4-specific CAR T-cell is preferred.

The skilled person is aware that CSPG4 is expressed on various tumors and its metastases such as melanomas, sarcomas, astrocytomas, gliomas, neuroblastomas, and leukemia. Hence, the CAR T-cells are generated according to Example 1 may be suitable for the treatment of melanomas, sarcomas, astrocytomas, gliomas, neuroblastomas, and leukemia.

Furthermore, the cell of the tumor may express tumor specific markers, which the skilled person is aware of. For example, the cell of the tumor may express CSPG4, CD19, CD20, CD22, CD30, CD269, CD138, HER2, EGFRvIII, PSMA, CEA, and/or MUC1. CD19, CD20, CD22 are exemplary targets on B cells. Further hematological targets may include BCMA (CD269), which may be expressed on in multiple myeloma. CD269 and/or CD138 are known targets in multiple myeloma; CD19 and/or CD30 are known targets in Hodgkin lymphoma; CD30 is a known target in anaplastic large cell lymphoma; CD19, CD20, CD22 and/or CD123 are known targets in acute lymphoblastic leukemia; CD19 is a known target in chronic lymphoblastic leukemia; CD19 and/or CD20 are known targets in Non-Hodgkin lymphoma. According to the present disclosure, the cell of the tumor preferably expresses CSPG4.

In general, tumors may be benign or malignant. Hence, the cell of the tumor may be a benign or a malignant tumor cell. Ideally the cell of the tumor is a malignant tumor cell.

The cell of the tumor may be a solid tumor cell or a hematopoietic malignant tumor cell. In case the tumor cell is a hematopoietic tumor cell, the hematopoietic tumor cell may be selected from a group consisting of an acute lymphoblastic leukemia cell, an acute myelogenous leukemia cell, a chronic lymphocytic leukemia cell, a chronic myelogenous leukemia cell, an acute monocytic leukemia Hodgkin's lymphoma cell, a Non-Hodgkin's lymphoma cell, a mantle cell lymphoma cell, and a multiple myeloma cell. Preferably, the tumor to be treated is a hematopoietic tumor (hematological malignancy). Exemplary hematopoietic tumor may be acute lymphoblastic leukemia, acute myelogenous leukemia, chronic lymphocytic leukemia, chronic myelogenous leukemia, acute monocytic leukemia Hodgkin's lymphoma, Non-Hodgkin's lymphoma, mantle cell lymphoma, or multiple myeloma. Jurkat cells, as used in Example 2 herein, are an acute T cell leukemia cell line that is used to study human T cells in vitro. As demonstrated in Example 2, Jurkat cells can be readily loaded with superparamagnetic iron-based particles and detected by MRI.

In case the tumor cell is a solid tumor cell, the solid tumor cell is preferably a solid malignant tumor. Such an example of a the tumor cell may be selected from a group consisting of a melanoma cell, a bladder cancer cell, a breast cancer cell, a cervical cancer cell, a colon cancer cell, an endometrial cancer cell, an esophageal cancer cell, a gall bladder cancer cell, a gastric cancer cell, a glioblastoma cell, a head and neck cancer cell, a hepatocellular carcinoma cell, a lung cancer cell, a mesothelioma cell, a neuroendocrine cancer cell, a ovarian cancer cell, a pancreatic cancer cell, a prostate cancer cell, a renal cell carcinoma cell, and a sarcoma cell. In line with the presented data in Fig. 4, the tumor cell is preferably a melanoma cell.

Preferably, the tumor to be treated is a solid malignant tumor. Exemplary solid malignant tumor may be melanoma, bladder cancer, breast cancer, cervical cancer, colon cancer, endometrial cancer, esophageal cancer, gall bladder cancer, gastric cancer, glioblastoma, head and neck cancer, hepatocellular carcinoma, lung cancer, mesothelioma, neuroendocrine cancer, ovarian cancer, pancreatic cancer, prostate cancer, renal cell carcinoma or sarcoma. In accordance with Example 1, the tumor to be treated is preferably melanoma.

A CAR T-cell containing superparamagnetic iron-based particles (a "loaded CAR T-cell") is also disclosed in one aspect of the disclosure. Said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions.

Preferred embodiments regarding the CAR T-cell, the superparamagnetic iron-based particles, the reduced cytokine release, the tumor and/or the unloaded CAR T-cell are described herein and apply equally to a CAR T-cell containing superparamagnetic iron-based particles. For example, the superparamagnetic iron-based particle may have a hydrodynamic diameter of at most 120 nm. It is preferred that the hydrodynamic diameter is preferably at most 100 nm, more preferably at most 90nm, more preferably at most 80 nm, more preferably at most 75 nm. Conversely, the superparamagnetic iron-based particle may have a hydrodynamic diameter of at least 35 nm. Preferably, the superparamagnetic iron-based particle has a hydrodynamic diameter of at least 35 nm and at most 75 nm.

Furthermore, the use of a loaded CAR T-cell as an *in vitro* research tool is disclosed herein. Preferred embodiments regarding the CAR T-cell, the superparamagnetic iron-based particles, the reduced cytokine release, the tumor and/or the unloaded CAR T-cell are described herein and apply equally to the use of a loaded CAR T-cell. Said research tool may be used to study the loading procedure of T-cells, characteristics of superparamagnetic iron-based particles for loading, chemotaxis/homing, cell adhesion/diapedesis, and/or magnetic movability of cells.

Moreover, an *in vitro* method of generating a CAR T-cell containing superparamagnetic iron-based particles (a "loaded CAR T-cell") is disclosed herein. Said method comprises, preferably consists of, the steps:
a) introducing a CAR mRNA into a T-cell; and
b) loading the T-cell of step (a) with superparamagnetic iron-based particles
c) whereby the loaded CAR T-cell is generated.

Preferred embodiments regarding the CAR T-cell, and/or the superparamagnetic iron-based particles, are described herein and apply equally to the *in vitro* method of generating a CAR T-cell. For example, T cell may be a CD3+CD8+ or CD3+CD4+ α/β or γ/δ T cell. The skilled person is aware of suitable CAR mRNA in order to generate a CAR T-cell. In some embodiment, the mRNA encodes a CAR specific for CSPG4, CD19, CD20, CD22, CD30, CD269, CD138, HER2, EGFRvIII, PSMA, CEA, or MUC. It is preferred that the CAR mRNA encodes the sequence of a CSPG4-specific CAR. The skilled person is also aware of suitable methods to introduce the CAR mRNA into the T-cell. For example, the CAR mRNA may be introduced by electroporation, lipofectamine and/or PEI. It is preferred that the CAR mRNA is introduced by electroporation. Exemplary data for CAR T-cell generation is provided in Example 1 herein.

Before the superparamagnetic iron-based particles are introduced into the CAR T-cell, the superparamagnetic iron-based particles are cleaned of excess molecules in a preceding step before step (b) by aqueous washing based on porous membrane filtration followed by an ultrasound treatment. Preferred embodiments regarding said preparatory step disclosed herein apply equally to the in vitro method of generating a CAR T-cell. Specifically, the ultrasound treatment may be performed with at least 1.25 kJ/mL and at most 10 kJ/mL of energy density.

The superparamagnetic iron-based particles of step (b) may have a hydrodynamic diameter of at most 120 nm, preferably at most 100 nm, more preferably at most 90nm, more preferably at most 80 nm, and even more preferably at most 75 nm. Preferably the superparamagnetic iron-based particles have a minimal hydrodynamic diameter of at least 35 nm.

As supported and exemplified by the methods section of **Example 1** herein, the superparamagnetic iron-based particles are preferably citrate coated. It is also preferred that the excess citrate is washed from the superparamagnetic iron-based particles by aqueous washing based on porous membrane filtration followed by an ultrasound treatment. If the superparamagnetic iron-based particles are citrate coated, the superparamagnetic iron-based particles are coated, washed and dried before step (b) of the *in vitro* method of generating a CAR T-cell containing superparamagnetic iron-based particles.

A method of treating a tumor in a patient with a CAR T-cell containing superparamagnetic iron-based particles (a "loaded CAR T-cell"), wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions, is also disclosed herein. Preferred embodiments regarding the CAR T-cell, and/or the superparamagnetic iron-based particles, the reduced cytokine release, the tumor, the cell of the tumor and/or the unloaded CAR T-cell are described herein and apply equally to the method of treating a tumor in a patient.

The invention is further described by the following embodiments:
1. A T-cell expressing a chimeric antigen receptor ('CAR T-cell') containing superparamagnetic iron-based particles ('loaded CAR T-cell') for use in treating a tumor, wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions.
2. The loaded CAR T-cell for use of embodiment 1, wherein the cytokine release comprises the release of TNFalpha, IFNgamma and/or IL-2, preferably wherein the cytokine release comprises at least two of TNFalpha, IFNgamma and/or IL-2, more preferably wherein the cytokine release comprises TNFalpha, IFNgamma and IL-2.
3. The loaded CAR T-cell for use of embodiments 1 or 2, wherein the reduced cytokine release is at least reduced by 20%, preferably at least 30%, more preferably at least 40%, and even more preferably at least 50%, compared to cytokine release by the unloaded CAR T-cell.
4. The loaded CAR T-cell for use of any of the preceding embodiments, wherein the reduced cytokine release is measurable by an *in vitro* assay, preferably wherein the *in vitro* assay is selected from an enzyme-linked immunosorbent assay (ELISA), an enzyme-linked immune absorbent spot (ELISpot), Fluorospot assay and a bead based immunoassay, more preferably wherein the *in vitro* assay is a bead based immunoassay or an ELISA, and even more preferably a bead based immunoassay.
5. The loaded CAR T-cell for use of any of the preceding embodiments, wherein the reduced cytokine release is measurable by an assay performed on patient blood samples, preferably wherein the assay is selected from an enzyme-linked immunosorbent assay (ELISA), and a bead based immunoassay, more preferably wherein the in vitro assay is an ELISA.
6. The loaded CAR T-cell for use of embodiment 5, wherein the reduced cytokine release is further measurable by monitoring the patient's body temperature and/or CRP level, preferably wherein the patient's body temperature remains below 38°C and/or the CRP level remains below 10 mg/l.
7. The loaded CAR T-cell for use of any of the preceding embodiments, wherein the reduced cytokine release comprises a reduced TNFalpha release and wherein the reduced TNFalpha release is at least reduced by 20%, preferably at least 30%, more preferably at least 40%, more preferably at least 50%, and most preferably at least 60%, compared to cytokine release by the unloaded CAR T-cell.
8. The loaded CAR T-cell for use of any of the preceding embodiments, wherein the reduced cytokine release comprises a reduced IFNgamma release and wherein the reduced IFNgamma release is at least reduced by 20%, preferably at least 30%, more preferably at least 40%, more preferably at least 50%, more preferably at least 60%, even more preferably at least 70%, more preferably at least 80%, and most preferably at least 90%, compared to cytokine release by the unloaded CAR T-cell.
9. The loaded CAR T-cell for use of any of the preceding embodiments, wherein the reduced cytokine release comprises a reduced IL-2 release and wherein the reduced IL-2 release is at least reduced by 20%, preferably at least 30%, more preferably at least 40%, more preferably at least 50%, even more preferably at least 60%, and most preferably at least 70%, compared to cytokine release by the unloaded CAR T-cell.
10. The loaded CAR T-cell for use of any of the preceding embodiments, wherein the reduced cytokine release results in a reduced risk of an excessive inflammatory immune reaction, preferably wherein the excessive inflammatory immune reaction is a cytokine storm.
11. The loaded CAR T-cell for use of any of the preceding embodiments, wherein the loaded CAR T-cell is a 2^{nd} generation CAR T-cell, preferably wherein said 2^{nd} generation CAR T-cell comprises a co-stimulatory domain and an intracellular signaling domain, wherein the co-stimulatory domain comprises sections of CD28 or 4-1BB, more preferably wherein the co-stimulatory domain comprises section of CD28, and even more preferably wherein the co-stimulatory domain comprises section of CD28 and the intracellular signaling domain comprises section of CD3ζ, thereby forming a CD28-CD3ζ CAR.
12. The loaded CAR T-cell for use of any of the preceding embodiments, wherein the loaded CAR T-cell comprises from 0.5 to 4 pg iron per cell (Fe/cell), preferably from 0.55 to 3.5 pg Fe/cell, more preferably from 0.6 to 3.3 pg Fe/cell, more preferably from 0.65 to 3.1 pg Fe/cell, more preferably from 0.7 to 2.9 pg Fe/cell, more preferably from 0.8 to 2.7 pg Fe/cell, more preferably from 0.9 to 2.6 pg Fe/cell, more preferably from 1 to 2.5 pg Fe/cell, and most preferably around 1 pg Fe/cell.
13. The loaded CAR T-cell for use of any of the preceding embodiments, wherein the loaded CAR T-cell is stimulated upon binding to a cell of the tumor by the CAR T-cell.
14. The loaded CAR T-cell for use of any of the preceding embodiments, wherein the loaded and the unloaded CAR T-cell exhibit cytokine production intracellularly.
15. The loaded CAR T-cell for use of any of the preceding embodiments, wherein the loaded CAR T-cell exhibits a reduced cytokine production upon binding to a cell of the tumor by the CAR T-cell compared to the unloaded CAR T-cell under the same conditions, preferably wherein the cytokine production of the loaded CAR T-cell is reduced by at most 30 %, more preferably at most 25%, more preferably at most 20%, more preferably at most 15%, more preferably at most 12%, more preferably at most 10%, and even more preferably at most 8%.
16. The loaded CAR T-cell for use of any of the preceding embodiments, wherein the loaded CAR T-cell is magnetically attractable, preferably wherein the loaded CAR T-cell is magnetically attractable to an organ of interest.
17. The loaded CAR T-cell for use of any of the preceding embodiments, wherein the loaded CAR T-cell is magnetically guidable, preferably wherein the loaded CAR T-cell is magnetically guidable to an organ of interest.
18. The loaded CAR T-cell for use of any of the preceding embodiments, wherein the loaded CAR T cell is magnetically attractable with a magnetic field strength from 0.01 Tesla to 7 Tesla, preferably 0.02 Tesla to 6 Tesla, more preferably 0.03 Tesla to 5 Tesla, more preferably 0.04 Tesla to 4 Tesla, more preferably 0.05 Tesla to 3 Tesla, more preferably 0.05 Tesla to 2 Tesla, more preferably 0.05 Tesla to 1.5 Tesla, more preferably 0.07 Tesla to 1.2 Tesla, more preferably 0.1 Tesla to 1.1 Tesla, more preferably 0.2 Tesla to 1.05 Tesla, more preferably 0.3 Tesla to 1 Tesla, more preferably 0.4 Tesla to 1 Tesla more preferably 0.5 Tesla to 1 Tesla, more preferably 0.6 Tesla to 1 Tesla, more preferably 0.7 Tesla to 1 Tesla, and even more preferably around 1 Tesla.
19. The loaded CAR T-cell for use of any of the preceding embodiments, wherein the loaded CAR T-cell targets a solid tumor, preferably wherein the CAR T-cell targets melanoma, sarcoma, astrocytoma, glioma, neuroblastoma.
20. The loaded CAR T-cell for use of any of the preceding embodiments, wherein the loaded CAR T-cell is a CSPG4-specific, a HER2-specific, a EGFRvIII-specific, a PSMA-specific, a CEA-specific, a MUC1-specific, a CD19-specific, a CD20-specific, a CD22-specific, a CD30-specific, a CD269-specific, and/or a CD138-specific CAR T-cell, preferably wherein the loaded CAR T-cell is a CSPG4-specific CAR T-cell.
21. The loaded CAR T-cell for use of any of the preceding embodiments, wherein the loaded CAR T-cell is a theranostic agent, preferably wherein the theranostic agent is visible by MRI and/or ultrasound, more preferably wherein the MRI and/or ultrasound allows imaging controlled therapy.
22. The loaded CAR T-cell for use of embodiment 21, wherein the theranostic agent is visible by MRI, preferably wherein the MRI allows imaging guided therapy.
23. The loaded CAR T-cell for use of any of the preceding embodiments, wherein the superparamagnetic iron-based particles have been cleaned of excess molecules by aqueous washing based on porous membrane filtration followed by an ultrasound treatment, preferably wherein the ultrasound treatment has been performed with at least 1.25 kJ/mL and at most 10 kJ/mL of energy density.
24. The loaded CAR T-cell for use of any of the preceding embodiments, wherein the superparamagnetic iron-based particles have been cleaned of excess molecules by aqueous washing steps based on porous membrane filtration.
25. The loaded CAR T-cell for use of any of the preceding embodiments, wherein the superparamagnetic iron-based particles have a hydrodynamic diameter of at most 120 nm, preferably at most 100 nm, more preferably at most 90nm, more preferably at most 80 nm, more preferably at most 75 nm; and/or wherein the superparamagnetic iron-based particles have a hydrodynamic diameter of at least 35 nm.
26. The loaded CAR T-cell for use of any of the preceding embodiments, wherein the unloaded CAR T-cell has not been loaded with superparamagnetic iron-based particles.
27. The loaded CAR T-cell for use of any of the preceding embodiments, wherein the unloaded CAR T-cell comprises at most 0.1 pg Fe/cell.
28. The loaded CAR T-cell for use of embodiments 12 or 26, wherein the amount of iron per cell is measurable by determining the cell number of CAR T-cells per volume and determining the amount of iron in a lysed sample of CAR T-cells, preferably wherein the amount of CAR T-cells is measurable by flow cytometry and/or the amount of iron is measurable by AES.
29. The loaded CAR T-cell for use of any of the preceding embodiments, wherein the superparamagnetic iron-based particles are selected from an iron oxide, iron (Fe), iron-cobalt, alnico, permalloy particles, preferably wherein the superparamagnetic iron-based particles are superparamagnetic iron oxide nanoparticles (SPIONs).
30. The loaded CAR T-cell for use of any of the preceding embodiments, wherein the superparamagnetic iron-based particles are superparamagnetic iron oxide nanoparticles (SPIONs) and wherein the iron oxide is selected from Fe₃O₄, γ-Fe₂O₃, CoFe₂O₄ or a mixture thereof, more preferably wherein the iron oxide is Fe₃O₄.
31. The loaded CAR T-cell for use of any of the preceding embodiments, wherein the superparamagnetic iron-based particles have been coated with citrate, oxalic acid, ascorbic acid, fatty acids and/or amino acids, preferably citrate.
32. The loaded CAR T-cell for use of any of the preceding embodiments, wherein the superparamagnetic iron-based particles have been coated with citrate following the protocol as described in Mühlberger et al. 2019 [19].
33. The loaded CAR T-cell for use of any of the preceding embodiments, wherein the superparamagnetic iron-based particles are detectable by MRI, preferably wherein the superparamagnetic iron-based particles are detected by MRI, more preferably wherein the T2^{∗} relaxation time of loaded CAR T-cells is reduced compared to the unloaded CAR T-cell under the same conditions, even more preferably wherein the T2^{∗} relaxation time is reduced by at least 30 % compared to the unloaded CAR T-cell under the same conditions, even more preferably 40% compared to the unloaded CAR T-cell under the same conditions, and most preferably at least 50% compared to the unloaded CAR T-cell under the same conditions.
34. The loaded CAR T-cell for use of any of the preceding embodiments, wherein the superparamagnetic iron-based particles inhibit the cytokine release from the loaded CAR T-cell.
35. The loaded CAR T-cell for use of any of the preceding embodiments, wherein the superparamagnetic iron-based particles have been introduced into the CAR T-cell by incubating the CAR T-cell with the superparamagnetic iron-based particles.
36. The loaded CAR T-cell for use of any of the preceding embodiments, wherein the superparamagnetic iron-based particles have been introduced into the CAR T-cell by incubation of the CAR T-cells with superparamagnetic iron-based particles after electroporation, by treatment with an effective amount of lipofectamine and/or by treatment with an effective amount of PEI, preferably by incubation of the CAR T-cells with superparamagnetic iron-based particles after electroporation.
37. The loaded CAR T-cell for use of any of the preceding embodiments, wherein the cell of the tumor expresses the antigen CSPG4, CD19, CD20, CD22, CD30, CD269, CD138, HER2, EGFRvIII, PSMA, CEA, and/or MUC1, preferably wherein the cell of the tumor expresses CSPG4.
38. The loaded CAR T-cell for use of any of the preceding embodiments, wherein the cell of the tumor is a malignant tumor cell.
39. The loaded CAR T-cell for use of any of the preceding embodiments, wherein the cell of the tumor is a hematopoietic malignant tumor cell.
40. The loaded CAR T-cell for use of embodiments 38 or 39, wherein the tumor cell is a hematopoietic tumor cell, preferably wherein the hematopoietic tumor cell is selected from a group consisting of an acute lymphoblastic leukemia cell, an acute myelogenous leukemia cell, a chronic lymphocytic leukemia cell, a chronic myelogenous leukemia cell, an acute monocytic leukemia Hodgkin's lymphoma cell, a Non-Hodgkin's lymphoma cell, a mantle cell lymphoma cell, and a multiple myeloma cell.
41. The loaded CAR T-cell for use of any of the preceding embodiments, wherein the cell of the tumor is a solid tumor cell, preferably wherein the solid tumor cell is a solid malignant tumor cell.
42. The loaded CAR T-cell for use of embodiment 41, wherein the tumor cell is selected from a group consisting of a melanoma cell, a bladder cancer cell, a breast cancer cell, a cervical cancer cell, a colon cancer cell, an endometrial cancer cell, an esophageal cancer cell, a gall bladder cancer cell, a gastric cancer cell, a glioblastoma cell, a head and neck cancer cell, a hepatocellular carcinoma cell, a lung cancer cell, a mesothelioma cell, a neuroendocrine cancer cell, a ovarian cancer cell, a pancreatic cancer cell, a prostate cancer cell, a renal cell carcinoma cell and a sarcoma cell, preferably wherein the tumor cell is a melanoma cell.
43. The loaded CAR T-cell for use of any of the preceding embodiments, wherein the tumor is a benign or malignant tumor, preferably a malignant tumor.
44. The loaded CAR T-cell for use of any of the preceding embodiments, wherein the tumor is a solid tumor, preferably wherein the solid tumor is a solid malignant tumor.
45. The loaded CAR T-cell for use of embodiment 44, wherein the tumor is selected from a group consisting of melanoma, bladder cancer, breast cancer, cervical cancer, colon cancer, endometrial cancer, esophageal cancer, gall bladder cancer, gastric cancer, glioblastoma, head and neck cancer, hepatocellular carcinoma, lung cancer, mesothelioma, neuroendocrine cancer, ovarian cancer, pancreatic cancer, prostate cancer, renal cell carcinoma and sarcoma, preferably wherein the tumor is melanoma.
46. The loaded CAR T-cell for use of any of embodiments 1-43, wherein the tumor is a hematopoietic tumor (hematological malignancy), preferably wherein the hematopoietic tumor is selected from a group consisting of acute lymphoblastic leukemia, acute myelogenous leukemia, chronic lymphocytic leukemia, chronic myelogenous leukemia, acute monocytic leukemia Hodgkin's lymphoma, Non-Hodgkin's lymphoma, mantle cell lymphoma and multiple myeloma.
47. A CAR T-cell for use in treating a tumor, wherein said CAR T-cell has been loaded with superparamagnetic iron-based particles, wherein said CAR T-cell exhibits a reduced cytokine release upon binding to a cell of the tumor by the CAR T-cell compared to CAR T-cell which is not loaded with superparamagnetic iron-based particles under the same conditions.
48. A CAR T-cell containing superparamagnetic iron-based particles (a "loaded CAR T-cell"), wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions.
49. The CAR T-cell of embodiment 48, wherein the superparamagnetic iron-based particle has a hydrodynamic diameter of at most 120 nm, preferably at most 100 nm, more preferably at most 90nm, more preferably at most 80 nm, more preferably at most 75 nm; and/or wherein the superparamagnetic iron-based particle has a hydrodynamic diameter of at least 50nm, preferably wherein the superparamagnetic iron-based particle has a hydrodynamic diameter of at least 35 nm and at most 75 nm.
50. The CAR T-cell of embodiment 48 or 49, wherein the loaded CAR T cell is defined in any of embodiments 11-22, the unloaded CAR T-cell is defined in any of embodiments 26-27, the amount of iron is defined in embodiment 28, the superparamagnetic iron-based particles are defined in any of embodiments 23-25 and 29-36, the cell of the tumor is defined in any of embodiments 37-42, and/or wherein the tumor is defined in any of embodiments 43-45.
51. Use of a loaded CAR T-cell according to any of embodiments 48-50 as an *in vitro* research tool.
52. An *in vitro* method of generating a CAR T-cell containing superparamagnetic iron-based particles (a "loaded CAR T-cell") comprising, preferably consisting of, the steps
   a. introducing a CAR mRNA into a T-cell; and
   b. loading the T-cell of step (a) with superparamagnetic iron-based particles whereby the loaded CAR T-cell is generated.
53. The method of embodiment 52, wherein the loaded CAR T-cell is defined in any of embodiments 11-21, the T-cell cell of step (a) is defined in any of embodiments 25-26, the amount of iron is defined in embodiment 27, and/or the superparamagnetic iron-based particles are defined in any of embodiments 22-23, 28-36.
54. The method of embodiment 52 or 53, wherein the T cell is a CD3+CD8+ or CD3+CD4+ α/β or γ/δ T cell.
55. The method of any of embodiments 52-54, wherein the mRNA encodes a sequence of the CAR specific for CSPG4, CD19, CD20, CD22, CD30, CD269, CD138, HER2, EGFRvIII, PSMA, CEA, or MUC1, preferably wherein the CAR mRNA encodes the sequence for the CSPG4-specific CAR.
56. The method of any of embodiments 52-55, wherein the CAR mRNA is introduced by electroporation, lipofectamine and/or PEI, preferably by electroporation.
57. The method of any of embodiments 52-56, wherein the superparamagnetic iron-based particles are cleaned in a preceding step of excess molecules by aqueous washing based on porous membrane filtration followed by an ultrasound treatment, preferably wherein the ultrasound treatment is performed with at least 1.25 kJ/mL and at most 10 kJ/mL of energy density.
58. The method of any of embodiments 52-57, wherein the superparamagnetic iron-based particles have a hydrodynamic diameter of at most 120 nm, preferably at most 100 nm, more preferably at most 90nm, more preferably at most 80 nm, more preferably at most 75 nm; and/or wherein the superparamagnetic iron-based particles have a hydrodynamic diameter of at least 35 nm.
59. The method of any of embodiments 52-58, wherein the superparamagnetic iron-based particles are citrate coated and wherein excess citrate is cleaned by aqueous washing steps based on porous membrane filtration.
60. A method of treating a tumor in a patient with a CAR T-cell containing superparamagnetic iron-based particles (a "loaded CAR T-cell"), wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ("unloaded CAR T-cell') under the same conditions.
61. The method of embodiment 60, wherein the cytokine release is defined in any of embodiments 2-10, wherein the loaded CAR T cell is defined in any of embodiments 11-22, the unloaded CAR T-cell is defined in any of embodiments 26-27, the amount of iron is defined in embodiment 28, the superparamagnetic iron-based particles are defined in any of embodiments 23-25 and 29-36, the cell of the tumor is defined in any of embodiments 37-42, and/or wherein the tumor is defined in any of embodiments 43-46.
62. The CAR T-cell of embodiment 48, 49, or 50 obtained by methods of any of embodiments 52 -59.

### Figure legends

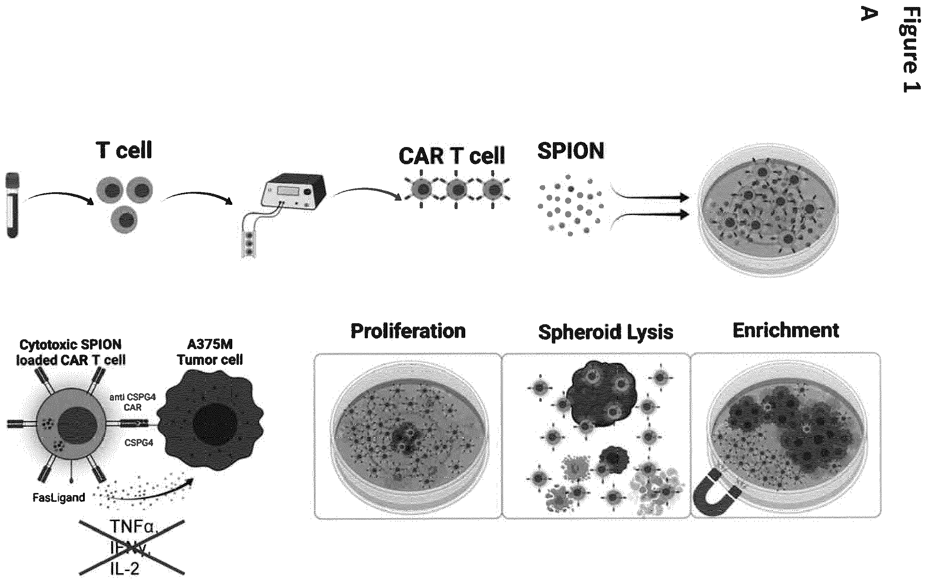

**Figure 1A****. Overview of invention.** CAR T-cells are loaded with SPIONs for therapeutic use.

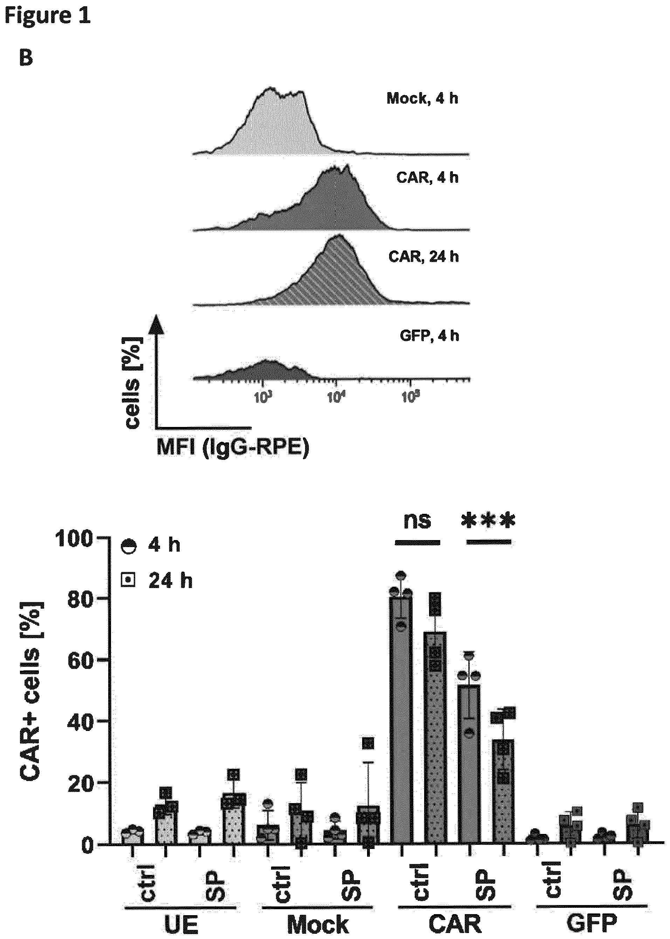

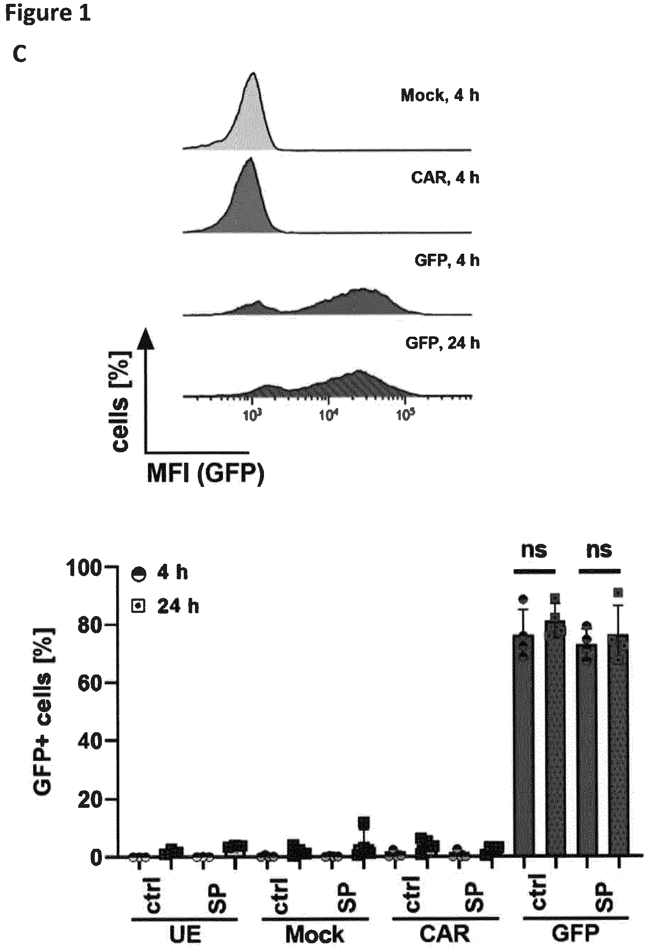

**Figure 1B** and C. Kinetics of CAR expression on T-cells. Isolated CD3+ T-cells of healthy donors were either left unelectroporated (UE), electroporated with no RNA (Mock) or received CSPG4-specific RNA via electroporation (CAR). As control for electroporation efficacy, RNA coding for GFP was transfected (GFP). T-cells were either incubated with 80 µg Fe/ml of SPIONs for 4h or with an equal amount of deionized H₂O. After 4 and 24 h, T-cells were stained for CAR and GFP expression and analyzed by flow cytometry. B) CAR-expression of T-cells. upper panel: MFI of one exemplary donor; lower panel: percentage of CAR+ T-cells after 4 h and 24 h. C) GFP-expression of cells. upper panel: mean fluorescence intensity (MFI) of one exemplary donor; lower panel: percentage of GFP+ T-cells after 4 h and 24 h. Statistical significances were calculated using a nested 1way ANOVA. ^{∗∗∗}p < 0.001, ns: not significant.

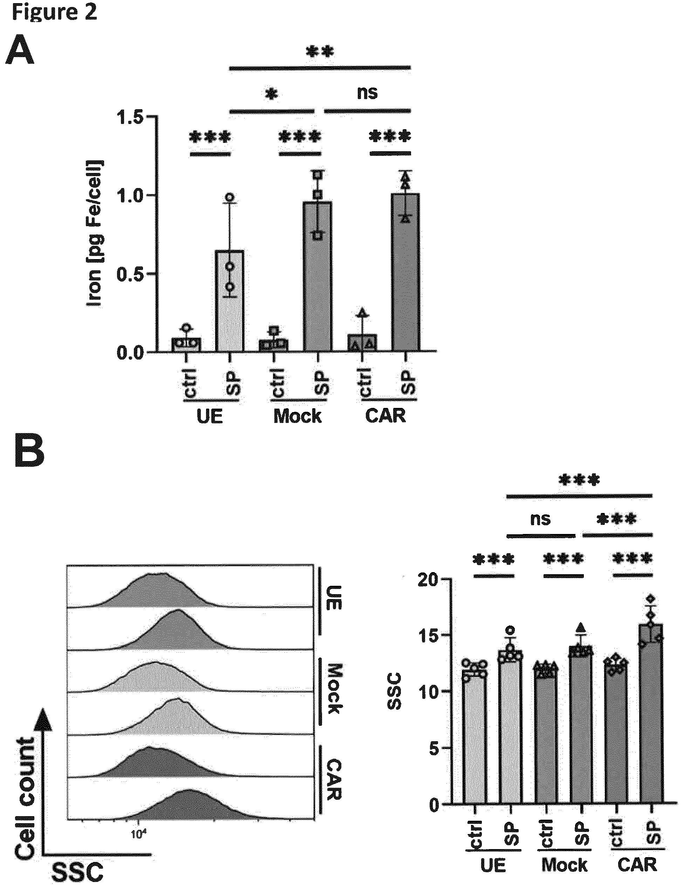

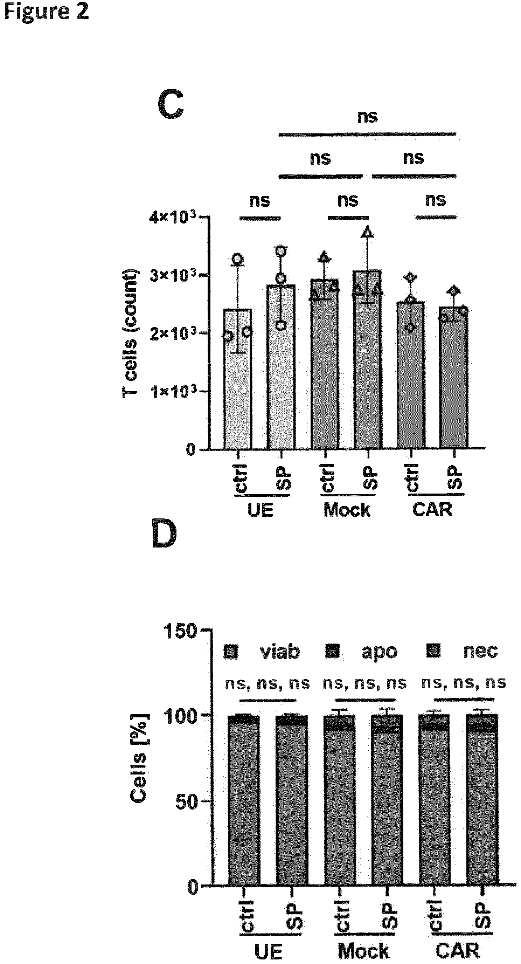

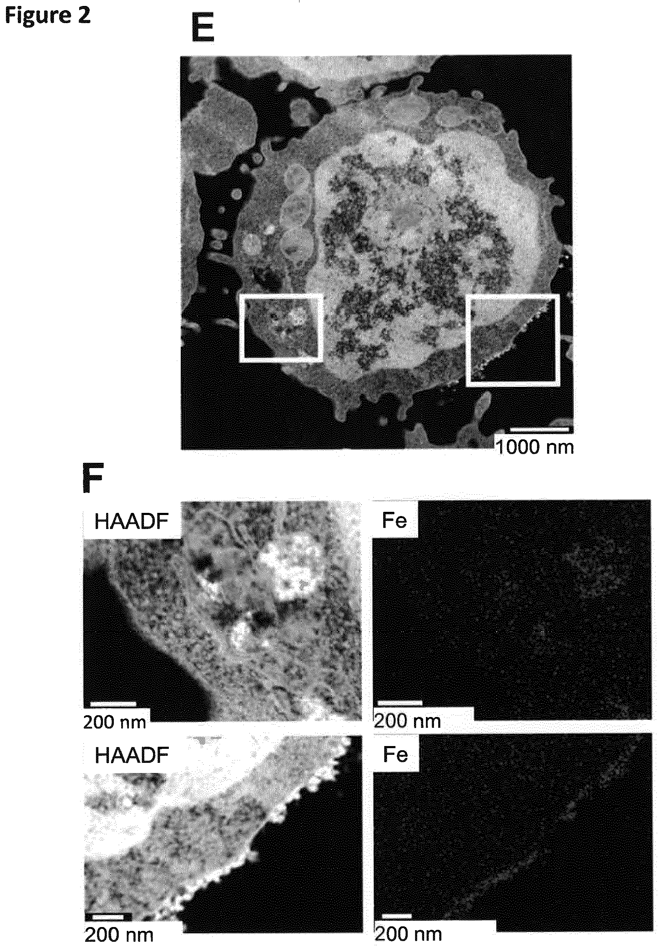

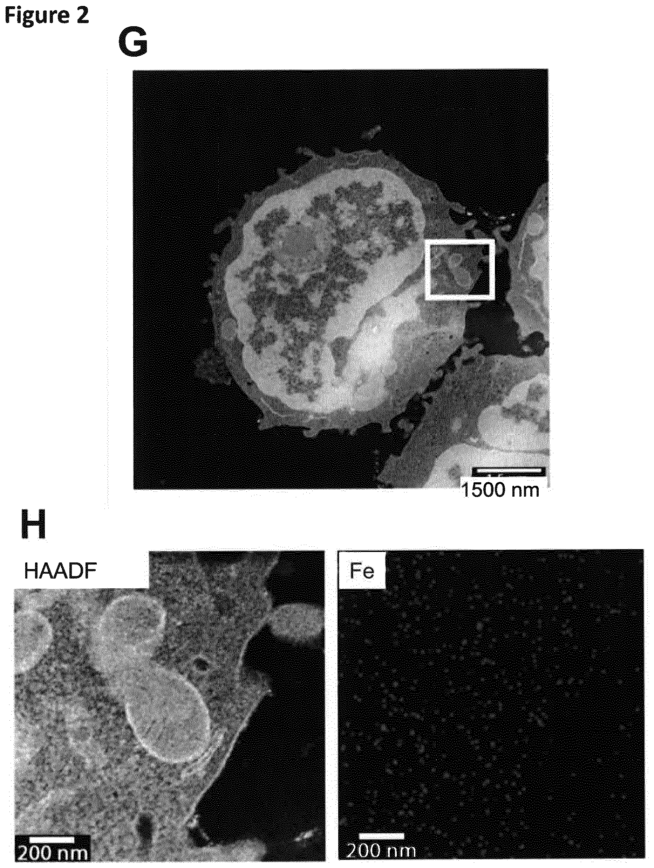

**Figure 2****. Nanoparticle uptake and viability of CAR T-cells after SPION-loading.** Isolated CD3+ T-cells were either left unelectroporated (UE), mock electroporated (Mock) or were transfected with RNA encoding the CSPG4-specific CAR (CAR). T-cells were loaded with 80 µg Fe/ml SPIONs overnight. Additionally, a control was established in which T-cells received only H₂O. A) Iron content of the cells was determined by atomic emission spectroscopy. B) SSC Histogram of differently treated T-cells determined by flow cytometry (left) and their absolute values (right). C) Cell count D) and viability were determined by flow cytometry after staining with AxV and propidium iodide. AxV- and PI- cells were determined as viable, AxV+ and PI- as apoptotic and AxV+ and PI+ as necrotic T-cells. E,G) Scanning Transmission Electronic Microscopy (STEM) analysis under dark field z-contrast. CD3+ T-cells loaded with 80 µg Fe/ml SPIONs for 24h (E,F), H₂O treated cells served as controls (G,H). Inserts in the STEM pictures show the corresponding energy dispersive X-ray (EDX) mapping for iron (Fe) highlighted in green in the scanned areas (right) (F,H). Shown are the mean values with standard deviations. The white bar depicts 1 µm (E), 1.5 µm (G) or 200 nm (F,H). Significances (ns; non significant; ^{∗∗∗}p < 0.001) were calculated using a 2way ANOVA with a posthoc Tukey correction. ctrl: control; SP: SPIONs

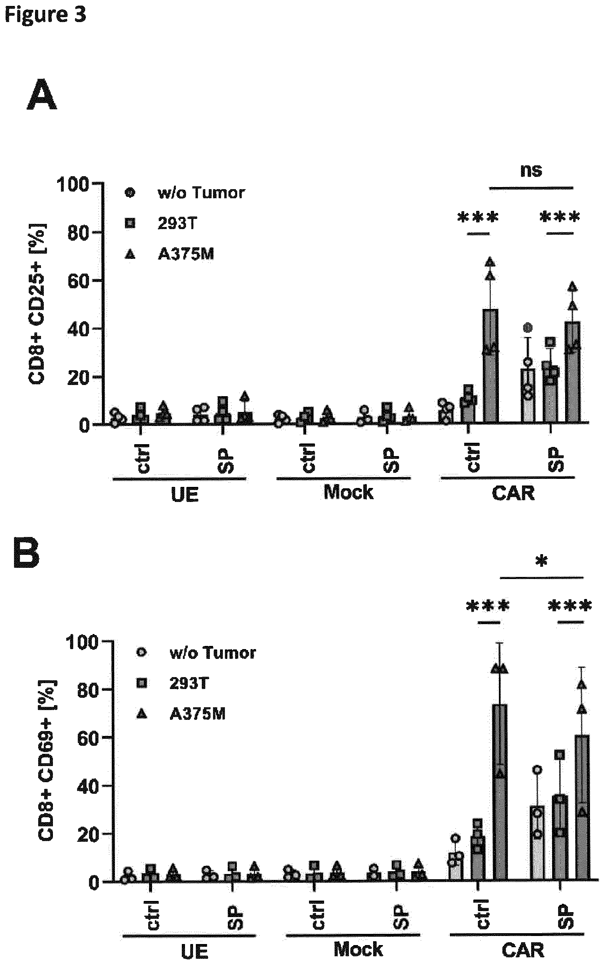

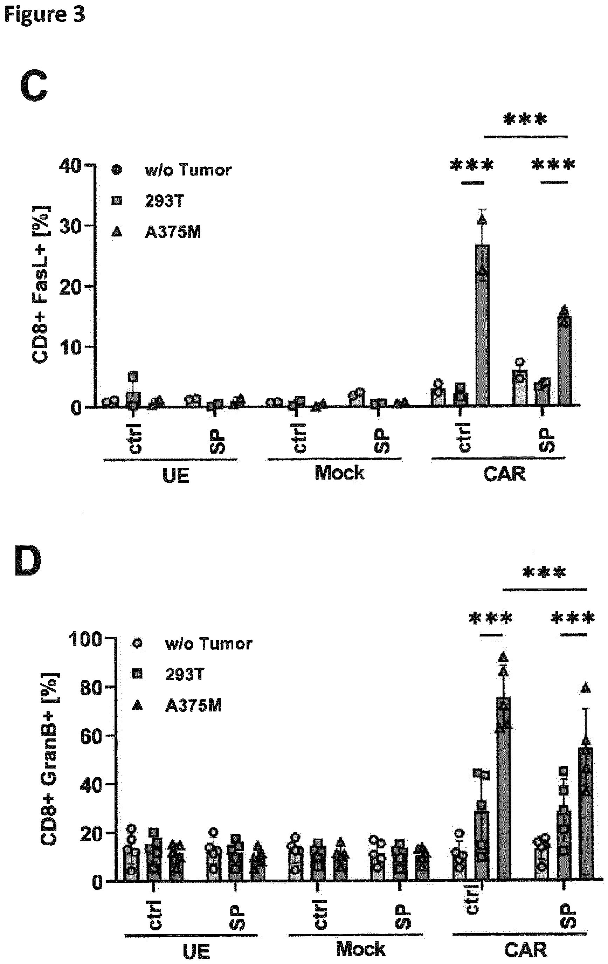

**Figure 3****. Effector functions of CSPG4-specific CAR T-cells after SPION-loading.** CD3+ T-cells were either left unelectroporated (UE), electroporated with no RNA (Mock) or were transfected with RNA encoding the CSPG4-specific CAR (CAR). Afterwards 50,000 T-cells were loaded with 80 µg Fe/ml SPIONs for 4 hours and then incubated with the same amount of A375M target cells or 293T control cells overnight for CD25 and CD69 and 60 h for Granzyme B and FasL, respectively. Then the cells were stained and analyzed by flow cytometry. Expression of A) CD25, B) CD69, C) FasL or D) Granzyme B on CD8 positive T-cells after tumor cell contact were analyzed. Significances were estimated using a 2way ANOVA with a posthoc Tukey test. ns: non significant, ^{∗}p < 0.05, ^{∗∗}p < 0.01, ^{∗∗∗}p < 0.001. ns: non significant; ctrl: control; SP: SPIONs; GranB: Granzyme B.
**Figure 4****. Cytokine production and release of CSPG4-specific CAR T-cells after SPION-loading.** T-cells were either left unelectroporated (UE), electroporated with no RNA (Mock) or were transfected with RNA encoding the CSPG4-specific CAR (CAR). Afterwards 50,000 T-cells were loaded with 80 µg/ml SPIONs for 4 hours and then incubated with the same amount of A375M target cells or 293T control cells overnight (A-E). Then, the cells were stained for CD8 and A) TNFα (A) or B) IFNy and analyzed by flow cytometry. C-E) Supernatant of T-cells co-incubated with A375M and 293T-cells was analyzed for the concentration of the secreted cytokines C) TNFα, D) IFNγ or E) IL-2. Significances were estimated using a 2way ANOVA with a posthoc Tukey test. ns, non significant, ^{∗}p < 0.05, ^{∗∗}p < 0.01, ^{∗∗∗}p < 0.001. ctrl: Control; SP: SPIONs.

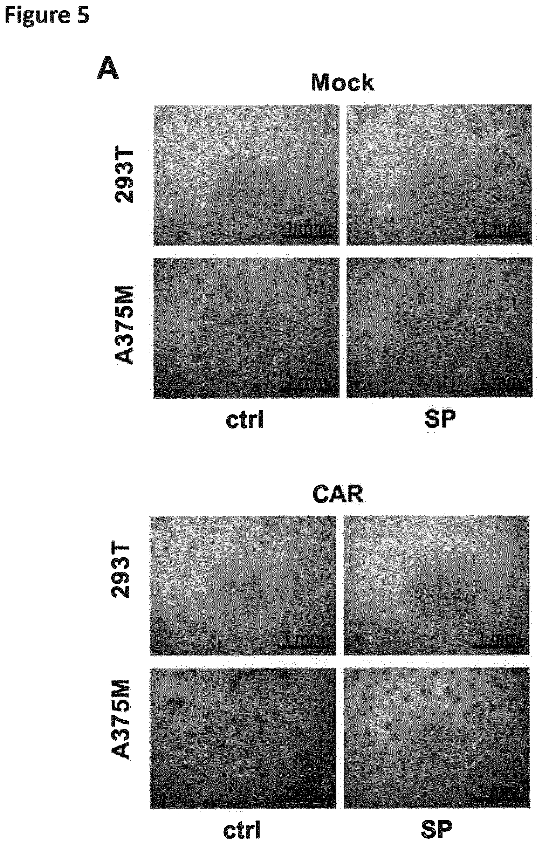

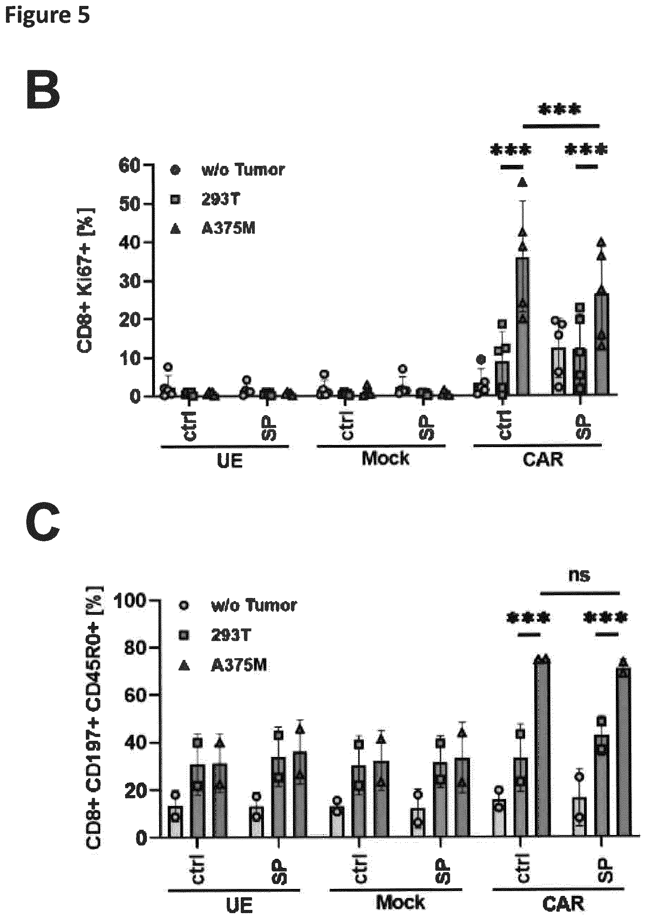

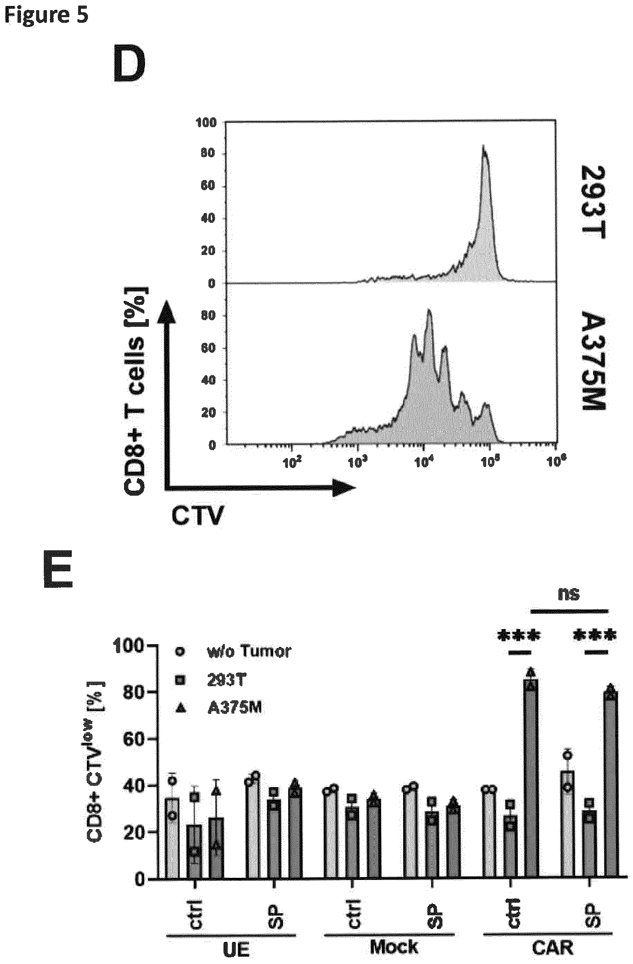

**Figure 5****. Proliferation and differentiation of CSPG4-specific CAR T-cells after SPION-loading.** Isolated CD3+ T-cells were either left mock electroporated (Mock) or were transfected with mRNA encoding the CSPG4-specific CAR (CAR). T-cells were loaded with SPION for 4 hours and then incubated in a 1:1 ratio with CSPG4-specific A375M melanoma cells or 293T-cells as controls. A) Proliferation clusters after 132 hours of incubation of T-cells with tumor cells analyzed in microscopy. B) Ki67+ as marker for cell division (t = 60 hours after antigen contact). C) Differentiation of CD8+ T-cells into central memory cells (CD45R0+/CD197+). D, E) Residual intracellular CellTrace^{™} Violet fluorescence provides information about number of cell divisions (t = 132 h after tumor cell contact). Significances were calculated using a 2way ANOVA with a posthoc Tukey test. ns: non significant, ^{∗∗}p < 0.05, ^{∗∗∗}p < 0.001. The black bar indicates 1 mm. ctrl: Control; SP: SPIONs.
**Figure 6****. Tumor cell lysis induced by SPION-loaded T-cells.** A-C) CSPG4-specific SPION-loaded CAR T-cells were incubated over night with CFSE-stained CSPG4-positive A375M cells in a ratio of 1:20. Unelectroporated (UE), Mock electroporated T-cells (Mock) and CSPG4-negative 293T-cells served as controls. Then, supernatant was collected and adherent cells were harvested by trypsinization. Adherent cells were stained with AxV-Fitc and PI. Supernatant and adherent cells were analyzed in flow cytometry. A) MFI of CFSE+ T-cells in supernatant indicates T cell-tumor cell interaction; B) T cell count in supernatant; cells were identified by their morphology; C) Percentage of necrotic tumor cells were identified by their AxV+ PI+ positivity. D-E) Real-time monitoring of cell impedance by the xCelligence system reflects cell adherence and therefore cell viability. 293T and A375M cells were incubated with T-cells in a ratio of 1:20 for 96 h. Loss of impedance indicates loss of cell adherence and cell death. Shown is the cell index over time for A375M cells. E, F) Area under the curve of the cell index over time for 293T-cells (E) and A375M cells (F) were calculated using GraphPad Prism, quantifying the target cell lysis. Significances were calculated using a 2way ANOVA with a posthoc Tukey test. ns: non significant, ^{∗∗}p < 0.01, ^{∗∗∗}p < 0.001. ctrl: control; SP: SPIONs.

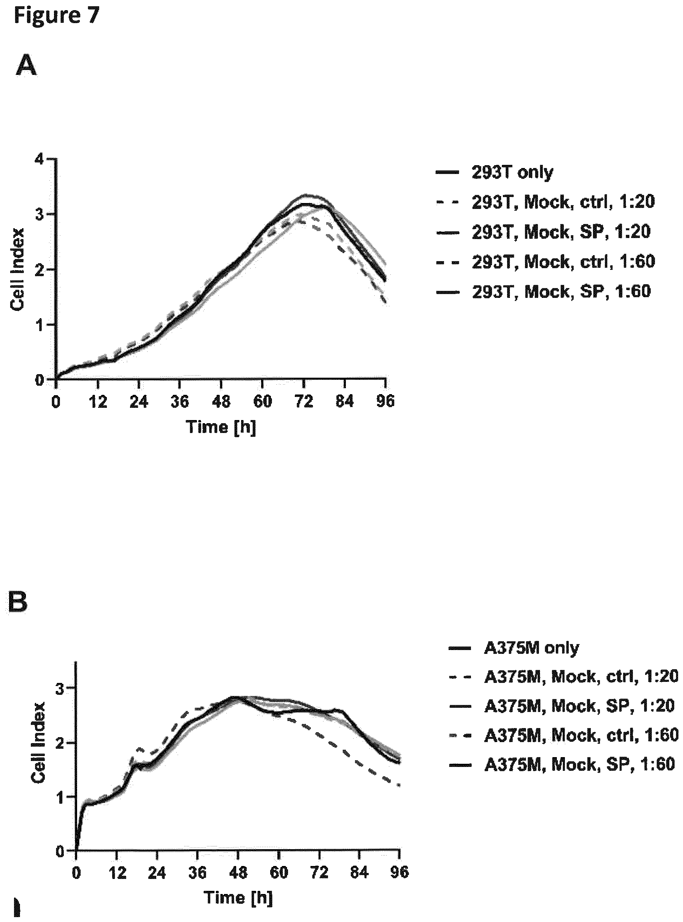

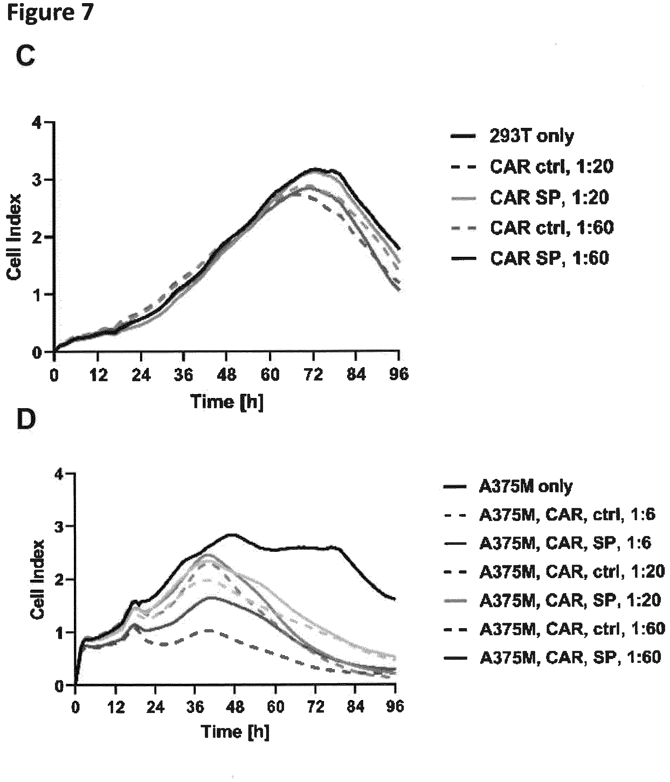

**Fig. 7****. Tumor lysis induced by SPION loaded T-cells.** T-cells received either no mRNA (Mock) or CAR mRNA via electroporation. Then the cells were loaded with 80 µg Fe/ml for 4 h or were treated with only H₂O as a control. Depicted are the cell index curves of Mock T-cells that were added to either A) 293T-cells or B) A375M cells at a 20:1 or 60:1 ratio. Shown are the cell index curves of CAR T-cells, which were added to either C) 293T-cells or D) A375M at a 6:1, 20:1 or 60:1 ratio.

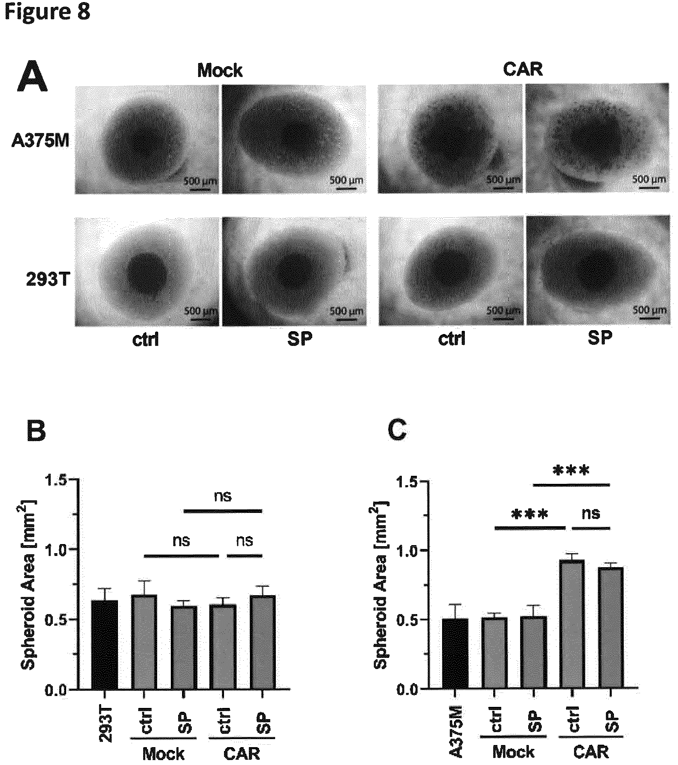

**Figure 8****. Tumor infiltration and spheroid lysis induced by SPION-loaded T-cells.** T-cells received no mRNA (mock) or CAR mRNA via electroporation. The T-cells were then loaded with 80 µg Fe/ml for 4 h or with H₂O as a control. A) The T-cells were then incubated with 3-dimensional 293T or A375M tumor cell spheroids for 36h at a ratio of 60:1, after which pictures were taken. Tumor size was then determined via ImageJ. B,C) determination of spheroid size after 36h of incubation. B) 293T-cells, C) A375M cells.
   Shown are the mean values with standard deviations. ns: non significant, ^{∗∗∗}p < 0.001. ctrl: Control; SP: SPIONs.

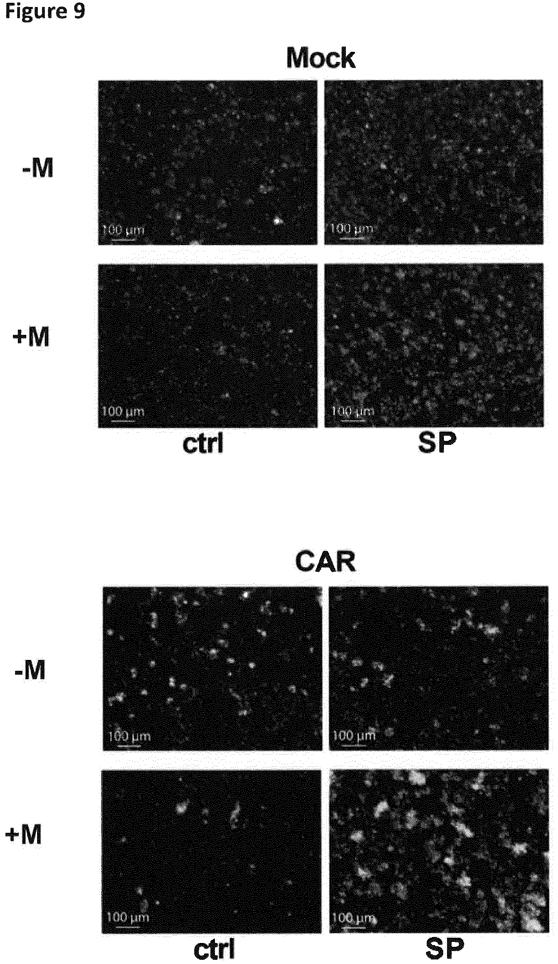

**Figure 9****: Local induction of tumor cell lysis by magnetically attracted SPION-loaded CAR T-cells.** Small magnets were placed under the wells, attracting SPION-loaded CAR T-cells. Vybrandt DiD stained T-cells (shown in red) were incubated 1:20 with Syto16 stained A375M cells (shown in green) for 16 h. Fluorescence images were taken at the initial positions of the magnets.-M: without a Magnet; +M: addition of a Magnet; ctrl: control; SP: SPIONs.

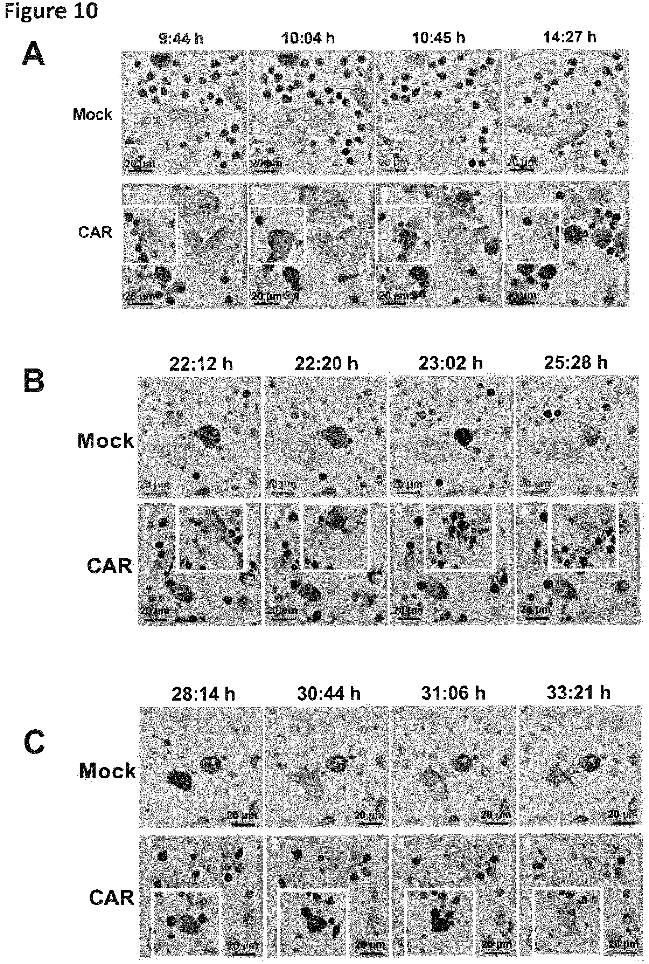

**Fig. 10****. Tumor lysis induced by SPION loaded T-cells.** T-cells received no mRNA (Mock) or CAR mRNA via electroporation. The T-cells were then loaded with 80 µg Fe/ml for 4 h. Subsequently, the cells were incubated with A375M tumor cells at a 60:1 ratio. The interaction between T-cells and A375M was analyzed in the Nanolive System, in which every 2 min pictures were taken. The black bar represents 20 µm. Lower rows: SPION-loaded CAR-T-cells. A-C) Inside of the white square (1) a viable tumor cell with T cell binding to it ("kiss of death"); (2) Initiation of apoptosis and tumor cell detachment; (3) apoptotic membrane blebbing; (4) remains of the lysed tumor cell can be seen. Upper rows: Non-electroporated, SPION-loaded T-cells served as controls. No tumor cell lysis was observed.
**Fig. 11****: Detection of SPION-loaded Jurkat T-cells in magnetic resonance imaging (MRI).** Jurkat cells were loaded with SPIONs for 4h. The iron content of the cells was analyzed using atomic emission spectroscopy and the cell count adapted so that the agarose phantoms contained 0.5, 1.0, 2.0 or 4.0 µg iron per ml. Then, phantoms were analyzed in MRI (T2^{∗}). A) T2^{∗} relaxation times (in ms) in MRI. B) Grey scale images.

### Examples

The following examples are intended to illustrate the invention further, but are not limited to it. The examples describe technical features, and the invention also relates to combinations of the technical features presented in this section.

### Example 1: Generation of SPION loaded CAR T-cells and their associated reduced cytokine release

### Synthesis and purification of SPIONs

Citrate coated SPIONs as described by Mühlberger et al. were synthetized and analyzed in triplicates regarding their basic physicochemical characteristics, like it has been done in [19] and slightly amended. Specifically, the superparamagnetic iron-based particles have been cleaned of excess molecules by aqueous washing steps based on porous membrane filtration and the superparamagnetic iron-based particles were not purified with acetone. The physicochemical features of three batches used in the actual experiments were very similar and in accordance with previous syntheses, proofing their reproducibility. The magnetic susceptibility at an iron concentration of 1mg/ml of the three batches was 4.08 ± 0.00, 4.12 ± 0.00 and 4.10 ± 0.00, the average hydrodynamic size [nm] in H₂O was 58 ± 0.1, 52 ± 0.1 and 53 ± 0.1, the polydispersity index (PDI) 0.143 ± 0.005, 0.151 ± 0.08 and 0.152 ± 0.07 and the zeta potential [mV] at a pH of 7.3 was -48.5 ± 5, -53.7 ± 0.4 and -51.8 ±0.3.

### Loading of CAR T-cells with SPIONS

It has been recently shown that electroporation of T-cells with CSPG4-specific CAR RNA allows the expression of CAR on the surface of CD3+ T-cells against the CSPG4-expressing melanoma cell line A375M [9]. To find an optimal time point for the loading with SPIONs, the inventors investigated the CAR expression on the T-cells 4 h and 24 h after electroporation by flow cytometry. As control for transfection, cells were electroporated with mRNA encoding GFP in parallel. The number of GFP expressing cells was similar at 4 h and 24 h and SPION loading did not alter it (Figure 1 C). The number of CAR expressing cells, however, was reduced from 4 h to 24 h and even more after SPION loading (Figure 1 B). The inventors speculate that this might be due to endocytic processes induced by SPIONs and invagination of the plasma membrane, which contains the CAR receptor. Non-electroporated and mock electroporated cells did not show any expression of CSPG4-specific CAR and GFP.

### Nanoparticle uptake and viability of CAR T-cells after SPION-loading

Based on the data of CAR expression and earlier data on SPION loading, the inventors decided to load CAR T-cells 1 h after electroporation with 80 µg/ml SPIONs for 4 h before incubation of CAR T-cells with tumor cells. Non- and mock-electroporated cells served as controls. Atomic emission spectroscopy (AES) revealed for all non-loaded T-cells around 0.1 pg Fe/cell, which increased from 0.65 (UE) to 0.95 (Mock) and 1.01 (CAR-T-cells) **(****Figure 2A****).** For electroporated samples, iron uptake may have been facilitated by the generated pores in the plasma membrane. Electroporation or addition of lipofectamin has been shown to improve labelling of lymphocytes with iron oxide nanoparticles before. [25-27]. Cellular side scatter, a marker for nanoparticle uptake [28], confirmed the increased SPION uptake of electroporated cells. For all non-loaded cells, the inventors determined similar SSC values around 12, which increased after SPION loading to 13.7 (UE), 14.0 (Mock) and 15.9 (CAR), respectively (Fig. 2B). Staining with AxV and PI for detection of exposure of phosphatidylserine and plasma membrane integrity enabled the discrimination of viable (AxV-/PI-), apoptotic (AxV+/PI-) and necrotic cells (PI+) in flow cytometry. In contrast to former studies by the working group [18, 21] incubation of T-cells with SPIONs only slightly decreased cell count and viability (Figure 2C,D). This improvement of overall viability may be due to the addition of IL-7 in this study, which plays an essential role in lymphoid cell survival.

Since T-cells are small, non-phagocytic, low endocytic cells with high nucleus to cytoplasm ratio, loading of cells is rather difficult [29]. As the amount of internalized nanoparticles is dependent on cell size, T-cells are not able to take up as much as larger, antigen presenting cells [30-32]. SPION internalization is limited to internalization by receptor-mediated endocytosis, clathrin-independent pathways and macropinocytosis in activated T-cells. For receptor-mediated endocytosis by clathrin-coated vesicles, a nanoparticle diameter of around 50 nm seems to be optimal [33, 34], which was the size of our particles. The inventors investigated the uptake of SPIONs by CD3+ T-cells via scanning transmission electron microscopy (STEM) dark field imaging and EDX analysis (

Figure 2 E-H). In SPION treated cells, STEM revealed single particles on the cells' plasma membrane and intracellularly in vesicles as well (Figure 2 E,F). The untreated control cells did not show any particles (

Figure 2 G,H). To be sure that the observed structures were SPIONs, EDX analysis on the respective regions was performed. The inventors identified SPIONs by their shape and Z-contrast, according to the scattering electrons. EDX mapping shows the cartography of the position of iron (

Figure 2 F,H). The inventors found a clear correlation between the position of the SPIONs and the EDX mapping. Random background was found in areas not containing particles in SPION-loaded samples or in controls. Remarkably, despite negative zeta potential of our SPIONs, tight attachment of the particles to the negatively charged plasma membrane with no spilling to unloaded cells was detected [18]. This is in contrast to others, who used 3-aminopropyl-triethoxysilane coated, positively charged SPIONs, which massively bound to the T cell surface but spilled over to unloaded cells [35].

### Effector functions of CSPG4-specific CAR T-cells after SPION-loading

Next, the antigen-specific activation of SPION-loaded CSPG4 specific CAR T-cells after contact with the CSPG4-expressing melanoma cell line A375M was analyzed. Non- and mock-electroporated and non-loaded T-cells served as controls. T cell responses towards CSPG4 positive A375 cells were compared with them towards CSPG4-negative 293T-cells. CD69 and CD25 as very early and later activation markers, and GranB and FasL as apoptosis inducing factors were analyzed [36]. In mock cells, hardly any cells expressing CD25, CD69, FasL or GranB were detectable. In CAR cells, the markers were generally slightly upregulated. As already published by Dörrie et al. the activation may be the result of intrinsic activity of the signaling modules within the CAR (i.e., CD28, CD3ζ) [37]. In the presence of CSPG4 expressing A375M cells, the percentage of CAR T-cells positive for the above mentioned markers were much higher compared to those incubated with CSPG4 negative 293T-cells. With SPION-loading, the percentage of CAR cells was slightly decreased in all cases **(****Figure 3****).** Concerning CD25 and CD69, the inventors found that SPION-loading of T-cells led to an increased unspecific CAR T cell activation in T-cells alone and in the presence of 293T-cells **(**Figure 3 A,B). In contrast, the amount of cells expressing FasL and GranB was similar, no matter if loaded with SPIONs or not (Figure 3 C,D).

### Cytokine production of CSPG4-specific CAR T-cells and reduced cvtokine release after SPION-loading

Overwhelming cytokine secretion by CAR T-cells is one of the limiting factors of CAR T cell therapy. Incubation of unloaded CAR T-cells with A375M cells led to a higher percentage of intracellularly pro-inflammatory cytokine (IFNγ, TNFα) producing cells, as determined by intracellular antibody staining. SPION-loading did not influence the number of CAR T-cells with intracellularly produced cytokines (Figure 4 A,B). But in contrast, the release of IFNγ, TNFα, and IL-2 determined by a bead-based Legendplex assay was significantly inhibited in the presence of SPIONs, where barely cytokines were secreted after antigen contact **(****Figure 4****,** C-E). This is likely to be assigned to the interaction of (iron oxide) nanoparticles with ion channels [38, 39]. A reduced cytokine release is a very surprising and unexpected finding as SPIONs are not known to reduce the cytokine release in CAR T-cells. CAR-T-cells usually induce pyroptosis, an inflammatory form of cell death in the target cells. Pyroptosis is characterized by release of damage-associated molecular patterns (DAMPs: ATP, HSPs, HMGB1) from dying cells. Pyroptosing tumor cells trigger macrophages to massively release pro-inflammatory cytokines (IL-1β, IL-6), causing the cytokine release syndrome (CRS). Since SPIONs suppress release of inflammatory mediators from CAR-T-cells, the cell death phenotype in target cells might be less inflammatory and macrophages will be not excessively activated. As cytokine and DAMP release by dying tumor cells can elicit a cytokine storm caused by activated macrophages, a reduced cytokine release by the CAR-T-cells is expected to have a crucial impact on therapeutic use of SPION loaded CAR T- cells. In addition, it has been surprising that the production of cytokines was only mildly reduced, while the release was significantly reduced.

### Proliferation and differentiation of CSPG4-specific CAR T-cells after SPION-loading

When the inventors investigated T cell proliferation and differentiation, in brightfield microscopy the inventors detected T cell proliferation clusters only in CAR T-cells when incubated with A375M cells, whereas in the presence of SPIONs the clusters seemed to be smaller **(****Figure 5****,** A). Estimating cell division by reduction of Cell Tracker Violet (CTV) fluorescence, high amounts of cells with low CTV fluorescence for CAR T-cells co-incubated with A375M were found, no matter if non-loaded or SPION-loaded **(****Figure 5****,** D-E). In line with these findings, the percentage of non-loaded cells, which were positive for the proliferation marker Ki67, was increased after incubation with A375M cells, while loading with SPIONs decreased it. Again, SPIONs slightly induced unspecific proliferation in CAR T-cells **(****Figure 5****,** B). Additionally, the inventors analysed cells for expression of CD45R0 and CD197 to identify central memory T-cells. In the presence of A375M cells, percentage of central memory cells was increased compared to T-cells alone or T-cells incubated with 293T. SPION loading had no impact on differentiation in CAR T-cells **(****Figure 5****,** C).

### Tumor cell lysis induced by SPION-loaded T-cells

Next, the capability of SPION-loaded CAR T-cells to bind and lyse target cells was analysed (**Figure** ). When CAR T-cells were incubated with A375M, the amounts of free T-cells in supernatant were reduced compared to incubation with 293T-cells, independently from SPION loading **(****Figure** 6, A-B). Analyzing the percentage of necrotic tumor cells (Ax+PI+ cells) after incubation with T-cells, the inventors found only 23.4 % and 26.2 % necrotic cells when incubated with 293T-cells (unloaded or SPION-loaded) versus 52.8 % and 56.8 % when incubated with A375M cells (unloaded or SPION-loaded) (Figure 6 C). Impedance measurement was performed to analyze cell adherence of target cells as marker for cell viability. Increase of impedance reflects increasing number of adherent cells, referred to as cell index (**Figure 6** **D**). To quantify the cell index over the total incubation time, the area under the curve (AUC) was assessed. A375M cells alone reached an AUC of 208.4, which was reduced to 107.2 or 118.3 with non-loaded or SPION-loaded CAR T-cells (**Figure** 6F). This effect was not detectable when CAR T-cells where incubated with 293T cells (Figure 6 E). With increasing CAR T-cell count the AUC steadily declined in the presence of A375M cells, whereas the addition of SPIONs reduced this effect insignificantly. Interestingly, the CAR T-cell ratio had no influence on the induction of tumor cell death at around 40 h, indicated by a continuous reduction of the cell index. However, the peak before lysis induction was at a lower cell index indirectly proportional to the added CAR T-cell number. Mock electroporated T-cells showed a delayed peak at around 50 h similar to A375M after 48 h (Figure 6 D). Additionally, different ratios of effector to tumor cells were also investigated on A375M and 293T-cells (**Figure 7**). The ability of SPION-loaded CAR T-cells of tumor infiltration was investigated using multicellular spheroids of A375M and 293T. After 36 h co-incubation time with 60:1 T-cells, A375M spheroids incubated with CAR T-cells showed non-homogenous margins and T-cells formed proliferation spots, indicating CAR T cell infiltration and subsequent cell lysis. Mock T-cells neither dissolved the tight spheroidal structure of A375M nor formed proliferation spots. The same occurred with mock or CAR T-cells incubated with 293T spheroids **(****Figure** 8A). Additionally, spheroid area was investigated via ImageJ. While the spheroid areas of A375M cells alone or mock-electroporated were at around 0.5 µm², addition of CAR T-cells increased the area to 0.93 µm², which was slightly reduced with SPION-loaded CAR T-cells to 0.87 µm². No significant differences in 293T cell spheroid area was detected, independently from the addition of mock or CAR, non-loaded or SPION-loaded T-cells (Figure 8 B,C). Finally, the ability of CAR T-cells to induce site-specific cell death after magnetic enrichment was evaluated **(****Figure** 9). Syto16-stained tumor cells (green, e.g. cells in top panel of Figure 9) were co-incubated overnight with DiD stained T-cells (red, red is only visible in 4^{th} panel of Figure 9 (Mock, +M, SP)) in wells with small magnets beneath. Analyzing co-localization, for SPION-loaded mock T-cells, the inventors found accumulation at the magnet position but no interaction with the tumor cells, as shown by separated green and red fluorescence. For CAR T-cells in contrast, magnets efficiently enriched SPION-loaded T-cells and fostered interaction between both cell types, as depicted by yellow fluorescence (yellow is only visible in 8^{th} panel of **Figure 9****,** (CAR, M+, SP)). Live cell imaging via non-invasive correlative epifluorescence confirmed antigen-specific lysis of A375M cells by SPION-loaded CAR T-cells (**Figure** 10). After 9:44 h, the CAR T-cells started to associate with the A375M tumor cell ("kiss of death"), which formed apoptotic blebs (10:45 h) and subsequently detached (14:27 h) (**Figure** 10A, lower panels). Non-electroporated SPION-loaded CAR T-cells did not induce apoptosis in the given time (**Figure** 10, upper panels).

### Conclusion

CAR T cell therapy of solid tumors has been difficult due to insufficient tumor infiltration and severe off-target toxicity. The modulation of effector T-cells can improve adoptive T cell therapy. In this study, CAR T-cells with citrate-coated SPIONs were functionalized to enable their magnetic guidance. The inventors not only achieved the tight attachment of SPIONs on the plasma membrane but also inside the T-cells in vesicles. After contact with their specific antigens, the magnetized CAR T-cells fulfilled their normal immune responses: upregulation of activation markers, proliferation and differentiation, expression of apoptosis-inducing factors, infiltration into tumor spheroids and, importantly, lysis of tumor cells. Remarkably, despite cells intracellularly produced cytokines, they were not able to release them, thus likely preventing cytokine release syndrome when applied *in vivo,* which is a known severe complication of CAR T-cell therapy. With magnetic navigation, the inventors were able to control the area of action to induce a site-directed tumor cell death. Summarizing, SPION-loading of CAR T-cells represents not only a possibility to increase their efficacy and simultaneously to reduce systemic side effects by magnetic targeting, but also provides the opportunity of monitoring the cells in magnetic resonance imaging for image-guided tumor therapy.

### Materials and methods

### Materials

H₂O used for all experiments was prepared in-house employing the Merck Milli-Q Direct water purification system (Darmstadt, Germany). Falcon cell strainer 40 µm and 70 µm were bought from Corning by Life Sciences (Corning, NY, USA) and LabSolute (Renningen, Germany). Cell culture plates were obtained from TPP (Trasadingen, Switzerland). Muse^{®} Count & Viability Assay Kit was purchased from Merck (Darmstadt, Germany). Fresenius Kabi (Bad Homburg, Germany) delivered Ringer's solution. Hoechst 33342 (Hoe), 1,1'dimethyl-3,3,3',3'-tetramethylindodicarbocyanine iodide (DiIC₁(5)), Penicillin-Streptomycin solution 5000 U/ml, L-glutamine (200 mM), Gibco RPMI medium 1640 and GlutaMAX^{™} supplement was acquired from Thermo Fisher Scientific (Waltham, MA, USA). Fetal calf serum (FCS) and Amphotericin B were acquired from Biochrom (Berlin, Germany). SYTO^{™} 16 Green Fluorescent Nucleic Acid Stain and Vybrandt^{™} DiD Cell-Labeling solution were bought from Thermo Fisher Scientific as well. Sigma-Aldrich (Taufkirchen, Germany) provided propidium iodide (PI) and phosphate-buffered saline (PBS). ImmunoTools (Friesoythe, Germany) delivered recombinant human interleukin 2 (IL-2) and recombinant human (IL-7), as well as Annexin A5 (AxV)-fluorescein isothiocyanate (FITC) and Allophycocyanin (APC) conjugate. Stemcell Technologies (Vancouver, BC, Canada) provided the ImmunoCult^{™} Human CD3/CD28/CD2 T cell Activator. Human CD3 Fab-TACS Gravity Kit was purchased from IBA (Goettingen, Germany). The nitric acid 65 % was delivered by Carl Roth (Karlsruhe, Germany). S-Monovettes 10 ml 9 NC were obtained from Sarstedt (Nuembrecht, Germany). BD Insyte-W intravenous cannula, 16GA were delivered from BD (Haryana, India). BioLegend (San Diego, CA, USA) provided PerCP/Cy5.5 anti-human CD4, Pacific Blue CD8a, APC anti-human CD8a, PE anti-human CD25, FITC anti-human CD69, PE anti-human Ki67, Pacific Blue^{™} Granzyme B, PE anti-human CD178, APC anti-human CD45R0, PE anti-human CD197, Zombie UV Fixable Viability Kit, Brefeldin A Solution (1,000X) and LEGENDplex^{™} Human CD8/NK Panel (13-plex). APC Mouse IgG1, kappa, Isotype, Ctrl (FC), APC anti-human CD38, APC Mouse IgG1, kappa isotype Ctrl (FC, APC anti-human CD45RO, APC Mouse IgG2a, kappa isotype Ctrl (FC), PerCP/Cy5.5 Mouse IgG2b, kappa isotype Ctrl, Pacific Blue Mouse IgG1, kappa, isotype Ctrl, PE anti-human HLA-DR, PE Mouse IgG2b, kappa isotype Ctrl, PE anti-human, CD197 (CCR7), PE Mouse IgG2a, kappa, isotype ctrl (FC). FITC mouse Anti-human CD3, Pacific Blue mouse anti-human CD8 antibody, Pacific Blue mouse IgG1, kappa, Isotype Control and FITC Mouse IgG1, kappa, Isotype Control were purchased from BD Pharmingen (San Jose, CA, USA). CellTrace^{™} Calcein Green, AM, CellTrace^{™} Violet and eBioscience^{™} Fixable Viability Dye eFluor^{™} 780 (FixV 780) was acquired from Invitrogen. Milteny Biotec (Bergisch Gladbach, Germany) delivered the Inside Stain Kit.

Agilent Technologies (Santa Clara, CA, USA) supplied the xCelligence RTCA SP, 4200 MP-AES and Dako Fluorescence Mounting Medium. Gallios flow cytometer and Kaluza Analysis Software (1.3, 2.1) were obtained from Beckman Coulter (Brea, CA, USA). Statistical analysis and graph creation was performed with GraphPad PRISM 9.0.2 from GraphPad Software, Inc. (San Diego, CA, USA).

For transmission electron microscopy (TEM), glutardialdehyde 25 % (for microscopy) in PO₄-buffer, ethanol (absolute), acetone (100 % anhydrous), hardener MNA, DPA, glycidyl ether (100 %), accelerator DMP 30, NaOH (1 M) was obtained from Carl Roth (Karlsruhe, Germany). The following equipment from these companies was used: Emerson (Saint Louis Missouri, United States) Branson 1200 ultrasonic machine, Leica (Wetzlar, Germany) Ultracut UCT, Zeiss (Oberkochen, Germany) TEM Leo 906, TRS Trondle (Moorenweis, Germany) CCD camera, ImageSP SysPROG.

Carl Roth (Karlsruhe, Germany) supplied Dimethylsulfoxid (M 78.13 g/mol), Glutardialdehyd 25 % (M 100.12 g/mol), 2.5 % GA in PO₄-buffer and Agarose standard. The Gallios flow cytometer was acquired from Beckmann Coulter (Brea, CA, USA), as well as Kaluza 1.0 and Kaluza Analysis Version 1.3 and 2.1. Hank's Balanced Salts Solution was purchased from BioWest (Nuaillé, France). Triton X-100 stock was acquired from Sigma (Missouri, USA). BioTracker, 650 Red Nuclear Dye EMD Milipore Corp (Burlington MA, USA). Iron reference standards (1 g/L) were purchased from Bernd Kraft GmbH (Duisburg, Germany). Webcraft GmbH (Gottmadingen, Germany) supplied Neodymium disc-shaped magnets (20 mm × 8 mm, approx. 200 mT, 5 mm × 5 mm, approx. 400 mT). 3D Cell Explorer-fluo and the Top-Stage Incubator were purchased from Nanolive SA (Tolochoenaz, Switzerland).

### Synthesis of SPIONs and physicochemical characterization

Three different charges of SPIONs were synthesized in-house based on an adjusted protocol of Elbialy [40]. Particles were sterilized by filtration through a 0.2 µm pore size filter. Absence of bacterial and endotoxin contaminations had been ensured by agar plate assays and an EndoZyme^{®} II - Recombinant Factor C Endotoxin Detection Assay (BioVendor R&D, Brno, Czech Republic), which was performed according to manufacturer protocol. The reaction was monitored for 90 min at 37 °C in 15 min intervals by recording the fluorescence at an excitation wavelength of 380 nm and an emission at 445 nm in a SpectraMax iD3 Plate reader (Molecular Devices, San Jose, USA). Spiking controls were carried out by adding a 5 µl of the highest standard to exclude assay interference.

Concerning their physicochemical features, SPION were analyzing regarding their size, iron content, magnetic susceptibility and zeta potential according to Mühlberger et al. [20]. Their iron content in mg/ml were investigated after diluting them 1:25 in deionized H₂O, liquefying them in 65 % nitric acid with atomic emission spectroscopy (AES), using Agilent 4200 MP-AES with an iron solution of 1000 mg Fe/I as an external standard (Bernd Kraft, Duisburg, Germany). SPION particles were diluted to the desired concentration with deionized water for all experiments.

### Isolation and cultivation of cells

Freshly drawn citrate anticoagulated blood was taken from informed volunteers (#257_14B). Leukocyte reduction chambers were obtained from the Transfusionsmedizin Erlangen (#60_21B). From both, T-cells were isolated using IBA CD3 Fab-TACS^{®} Isolation Kit, human, according to the manufacturer's instructions. In brief, whole blood was applied to gravity columns loaded with Fab CD3 antibodies to which the T-cells bound. After several washing steps the attached CD3+ T-cells were eluted via biotin. The linked CD3 antibody needed for the positive selection was discarded after centrifugation at 400 rcf for 10 min.

The T-cells were cultured in RPMI 1640 medium supplemented with 10 % heat-inactivated (HI) FCS 2 % penicillin-streptomycin-solution, 1 % L-glutamine, 1 % amphotericin B and 0.1 ng/ml IL-7, referred to as "T cell medium". A375M and 293T-cells were cultivated in RPMI 1640 medium supplemented with 10 % heat-inactivated (HI) FCS 2 % penicillin-streptomycin-solution, 1 % L-glutamine, 1 % amphotericin B, which will be denoted as "tumor cell medium". All cells were cultured in a humidified 5 % CO₂ atmosphere at 37 °C.

### Visualization of SPIONs using STEM and EDX

To evaluate the distribution of SPIONs on and in cells, STEM imaging and EDX spectrum imaging was conducted at a Cs-corrected Thermo Fisher Scientific (TFS) Spectra operating at 200 keV equipped with a SuperX G2 high-sensitivity X-ray spectrometer. The STEM micrographs were acquired using a camera length of 98 mm using an HAADF detector. EDX spectrum images were recorded with a dwell time of 50 µs - 100 µs and a screen current of approx. 0.250 nA. Processing and data evaluation were performed using the Velox software from TFS.

### Electroporation of T-cells with CSPG4-specific CAR mRNA and loading with SPIONs

Chondroitin sulfate proteoglycan 4 (CSPG4) specific CD3+ CAR T-cells were produced by mRNA electroporation as described previously [9]. Non-electroporated and Mock electroporated T-cells, which were electroporated but received no mRNA, were used as controls. Additionally, T-cells were also electroporated with GFP mRNA as a positive control for successful electroporation. Electroporation efficiency was analyzed 4 h and 24 h after electroporation, by staining T-cells for CAR expression for 30 min at 4 °C as well as analyzing GFP expression via flow cytometry. After electroporation, T-cells were seeded in 2 ml of T cell medium at a concentration of 1 × 10⁶ cells/ml and loaded with 80 µg SPIONs/ml in deionized H₂O for 4 hours, while the control T-cells received equal volumes of deionized H₂O without SPIONs. Cell viability was investigated by AxV-FITC, PI, Hoechst and DiI stain and analyzed in flow-cytometry. Iron uptake was quantified by atomic-emission-spectroscopy of 2 × 10⁶ loaded CAR T-cells after several washing steps to get rid of any excess iron. Mock-electroporated, non electroporated and unloaded T-cells served as controls.

### CAR T-cells activation and proliferation

5 × 10⁴ SPION-loaded or non-loaded T-cells per well were co-incubated in 200 µl T cell medium with 5 × 10⁴ A375M or 293T-cells in suspension in 96 well plates. Non electroporated and Mock-electroporated CAR T-cells served as controls.

Next day, supernatants were centrifuged at 400 g for 5 min and 50 µl samples frozen at - 80°C for analysis of cytokine secretion via LEGENDplex^{™} Human CD8/NK Panel (13-plex) according to the manufacturer's instructions. Cells were resuspended and 50 µl samples were taken and stained for CD4, CD8, CD25, and CD69 in PBS for 30 min at 4 °C, washed with PBS, fixated in 1 % formalin in PBS and analyzed in flow cytometry. To quantify apoptosis induction of SPION loaded CD8+ CAR T-cells, expression of Granzyme B and CD178 (FasL) was investigated. For that, triplicates of 5 × 10⁴ SPION-loaded CAR T-cells were co-incubated with 5 × 10⁴ A375M cells in suspension in 200 µl T cell medium for 60 h in a 96 well plate. 293T-cells, non electroporated and Mock electroporated T-cells, as well as unloaded T-cells served as controls. Subsequently, cells were resuspended and 50 µl samples were stained for CD4, CD8 and CD178 for 30 min at 4 °C. The cells were then fixated and permeabilized using the Inside Stain Kit according to the manufacturer's instructions. Subsequently the cells were stained for Granzyme B for 30 min at 4 °C and then analyzed by flow cytometry. For investigation of cytokine production cells were cultivated in the same manner but stained for CD4, CD8 extracellularly, fixated and permeabilized and stained intracellularly for IFNγ, TNFα.

After 60 h, cell proliferation was analyzed via Ki67 staining. 50 µl aliquots were taken and the cells were stained for CD4, CD8 and then fixated and permeabilized using the Inside Stain Kit according to the manufacturer's instructions. Ki67 was intracellularly stained and analyzed by flow cytometry.

For determination of cell division and cell differentiation, T-cells were stained with 5 µM CellTrace^{™} Violet (CTV) prior to seeding. After 120 h, 50 µl aliquots were taken and stained for CD3, CD8, CD45R0 and CD197 and then analyzed by flow cytometry to determine naive, central memory, effector memory and effector T-cells. Microscopy pictures of the T cell proliferation clusters were taken in parallel.

### Interaction of CAR T-cells with tumor cells

To analyze the interaction of the CAR T-cells with their CSPG4-expressing target cell line A375M, 1.2 × 10⁴ cells were seeded in a 24 well plate for two days. The human embryonic kidney cell line 293T was established as control, which does not express CSPG4. A375M and 293T-cells were stained with 2.5 µM CFSE according to the manufacturers protocol, media was removed and 2.4 × 10⁵ T-cells per well were added in T cell medium for 16 hours. Non electroporated and Mock electroporated T-cells served as controls. On the next day, the supernatant was removed, followed by detachment of the adherenT-cells by 0.5 % Trypsin. Cells in supernatant and sediment were stained with 2 µl/ml AxV-APC, 66.6 ng/ml PI, 20 nM Hoe [41] and analyzed by flow cytometry regarding viability, cell count and CFSE positive T-cells, indicating tumor binding.

### Impedance based and 3D analysis of tumor lysis by CAR T-cells

Tumor cell lysis was investigated via impedance based real-time cell analysis (xCelligence system). 7.5 × 10⁴ tumor cells per well were seeded in a 96 well Agilent E-plate for adherence. After 12 h, the SPION-loaded CAR T-cells were added in a ratio of 6:1, 20:1 or 60:1 in triplicates. 293T-cells, Mock electroporated T-cells, tumor cells alone, and unloaded T-cells served as controls. Controls with T cell medium only and T cell medium containing SPIONs were established to determine background noise.

For label-free observation of tumor cell lysis in Nanolive 3D Cell explorer-fluo, 1.5 × 10⁵ A375M cells were seeded in a 35 mm µ-dish for adherence. After 32 h, 6 × 10⁶ loaded CAR T-cells in T cell medium were added. The dish was kept inside a top-stage incubator ensuring stable humid conditions at 37 °C and 4 % CO₂ during the whole imaging process. For 30 hours every 2 min a 3D picture in 60 × magnification was taken, consisting of 96 layers and finally, a 3-dimensional picture of the interaction between T-cells and A375M tumor cells was recreated. Non-electroporated SPION-loaded T-cells were monitored as control.

### Magnetic accumulation of CAR T-cells on tumor cells

4 × 10⁴ tumor cells per well were seeded in a 24 well plate. After 48 h, tumor cells were stained with 5 µM Syto16 for 20 min at 37 °C. CAR T-cells were stained with 5 µM DiD at 37 °C for 20 min and washed with PBS before seeding them in a 20:1 ratio onto the tumor cells. To magnetically accumulate SPION-loaded T-cells, plates were incubated overnight on magnets, covering a part of each well. Cells without magnets, 293T-cells and Mock electroporated T-cells served as controls. Next day, the magnets were removed and the cells incubated for additional 6 h and subsequently analyzed by fluorescence microscopy.

### Spheroid infiltration by CAR T-cells

Multicellular cancer spheroids were generated by co-incubation of 1 × 10⁴ A375M cells with 1 × 10³ fibroblasts in an agarose-coated 96-well plate for five days. Spheroids cultured from 1 × 10⁴ 293T-cells and 1 × 10³ fibroblasts served as controls. SPIONs- loaded CAR T-cells were added in a ratio of 60:1 and co-incubated with the spheroid. After 36h microscopy pictures were taken. Unloaded T-cells and Mock-electroporated T-cells served as controls. The spheroid area was analyzed in ImageJ. To improve detection of the spheroid outline, a local threshold was performed based on the methods of Sauvola and Pietikainen [42].

### Purification of SPIONs via dialysis.

The dispersion of synthesized citrate-stabilized superparamagnetic iron oxide particles (Cit-SPIONs) was transferred into 25 cm long dialysis tubes with a molecular weight cut off (MWCO) of 10 kDa. The filled dialysis tubes were put into a 5 L beaker filled with 4.5 L of deionized H₂O (di. H₂O). Using a magnetic stirring bar and a magnetic stirrer, the particles were dialyzed under mild stirring for 1 h before the water was exchanged for fresh 4.5 L of di. H₂O. Exchanging the dialysis water every hour, the washing proceeded in total for 6 hours.

Afterwards, the dialyzed particles were transferred into a beaker and filtered through a 0.2 µm filter membrane to exclude any large agglomerates that might have formed. To break apart possible dimers of particles that might have generated during the dialysis step, filtered Cit-SPIONs were treated with an ultrasonic probe using at least 1.25 kJ/mL of energy density. The treated particles were cooled to room temperature using an ice bath and sterile filtered (0.2 µm-pore diameter syringe filter) for use in cell culture.

The purification method is in contrast to Mühlberger et al. [19].

### Purification of SPIONs by washing with acetone (Mühlberger et al.)

While Mühlberger purified the SPIONs solely by mechanically washing away byproducts which are insoluble in acetone, the described SPIONs herein were washed chemically during the dialysis step as the reaction byproducts are water-soluble. Hence, the SPIONS were cleaned of excess molecules by aqueous washing steps based on porous membrane filtration. Therefore, the described method provides a more efficient and reproducible cleaning of the SPION dispersion from reaction byproducts.

For application in cell culture, the dry stored particles were dissolved in water and sterile filtered through a 0.2 µm-pore diameter syringe filter.

### Example 2 - Visualisation of T-cells containing SPIONs in MRI

### Magnetic resonance imaging of SPION-loaded Jurkat cells

It is also shown herein that T-cells loaded with SPIONs can be made visible in MRI. This proof of principle is demonstrated in this example.

PCR tubes containing agarose phantoms were analyzed in magnetic resonance imaging 7T ClinScan (Bruker). The following parameters were used:

### T2^{∗} gre Morphology:

Sequence type: fl2d
Layer thickness: 0.2 mm
TR: 375ms
TE: 16ms
Flip Angle: 25°
Measurement time: 4:48 min.
Voxelsize: 0,208 × 0,208 × 0,2mm

### T2^{∗}Map:

Layer thickness: 0.5 mm
TR: 250 ms
TE 1-5: 3.19 ms; 7.18 ms; 11.17 ms; 15.16 ms; 19.15 ms
Measurement time: 42 min.
Voxelsize: 0,208 × 0,208 × 0,5mm

**Fig. 11A** shows a decreasing T2^{∗} relaxation time with increasing amounts of iron in the T-cells. The T2^{∗} relaxation time of unloaded T-cells is around 40 ms. When T-cells are loaded with 0.5, 1, 2 of 4 µg/ml of iron (Fe) the relaxation time reduces to around 25 ms, 20, 10 and 5 ms, respectively. Hence, for all tested concentrations the iron reduced the T2^{∗} relaxation time at least by 35%. This reduction in relaxation time can also be visually demonstrated in MRI as shown in **Fig. 11B****.** With increasing concentration of iron per cell, the cells appear darker in MRI. In other words, SPIONs are negative contrast agents, leading to dark signals in MRI. The visibility of SPIONs depends on their transversal (T2 and T2^{∗}) relaxivity. This proof-of principle example demonstrates that iron loaded T-cells can be visualized in MRI which harbors an additional advantage when used in patients. The iron-loaded cells can be specifically followed and generally visualized as they provide increased contrast compared to unloaded T-cells.

MRI visibility in patients also offers the control of magnetic targeting of SPION-loaded cells, as they are magnetically attractable and thereby guidable, and the control of therapy success. Specifically, SPIONs serve as theranostic agents as SPIONs can be detected via MRI while be targeted to the tumor. Also, the activation status of the T-cells might be visible since the T-cells start to proliferate after antigen contact and the signal will be diluted amongst the dividing daughter cells. At the same time the SPION loaded CAR T-cells are therapeutic as they cause lysis for the tumor cell but show a reduced cytokine release to lower the risk of a cytokine storm.

### Method: Loading of Jurkat cells

Jurkat cells were cultured in RPMI 1640 supplemented with 10 % FCS and 1 % L-glutamine. The cells were then loaded with 50 µg Fe/ml SPIONs at a concentration of 1×10⁶ cells/ml overnight. Controls were established receiving only H₂O. At the next day, the cells were washed twice with PBS to remove free SPIONs in the supernatant. The cells were then counted using the Muse^{®} Cell Analyzer according to the manufacturer's protocol. Two batches of 1×10⁶ Jurkat cells were then used to analyze the iron content per cell. The cells were centrifuged at 400 rcf for 5 min and all supernatant was removed. Then the cells were dried at 95 °C, 300 rpm for 30 min before they were lysed with 100 µl nitric acid at 95 °C, 750 rpm for 15 min. 900 µl H2O was added to the lysed cells, followed by atomic emission spectroscopy (AES), using an Agilent 4200 MP-AES with an iron solution of 1000 mg/l as an external standard. Triplicate measurements were performed at a wavelength of 371.993 nm, which were then averaged.

The cells were then diluted 125 µl PBS to reach the desired iron content in 0.2 ml PCR tubes. 2 % Agarose was prepared in beforehand and kept in a liquid stage at around 40 °C. After the addition of 125 µl of 2% agarose to the Jurkat cells, the PCR tubes were quickly placed in an ice bath to accelerate solidification and to ensure that the cells were permanently distributed as homogeneously as possible.

The tubes were kept in a wet and cold condition to prevent drying before and after the MRI measurements.

### Example 3 -Patient Treatment outline for magnetic targeting of SPION-loaded CAR T-cells

Based on the above cellular data on SPION loaded CAR T-cells and their technical effects, it is expected that said SPIONs loaded CAR T-cells can be used in patients. In doing so, the following steps are proposed, wherein steps 1.1 and 1.2 are partially based on *Wiesinger et at. 2019.[43]*

### 1. Preparation of SPION-loaded CAR T-cells for therapeutic use

### 1.1 Isolation and expansion of T-cells

Autologous T-cells are generated from patient's blood. In brief, on day 0 peripheral blood monocytes (PBMCs) are extracted from leukaphereses, using density centrifugation on Lymphoprep. Cells are expanded for nine days in X-vivo 15 medium in EVA bags with a starting concentration of 1×10⁶ cells/ml. OKT-3 (0.1 µg/ml) is added on day 0. IL-2 is added at day 0 (1000 IU/ml), day 2 (1000 IU/ml), day 5 (500 IU/ml) and day 7 (250 IU/ml). Cells are diluted at day 2 to 2×10⁵ cells/ml and at day 5 and 7 the volume of the X-vivo medium is doubled. At day 9, the cells are harvested and 3.24 ×10⁹ cells are electroporated with mRNA encoding the CSPG4-sepecific CAR.

### 1.2. Electroporation of T-cells

Following expansion, T-cells are washed and 150 × 10⁶ cells/ml are resuspendend in OptiMem and transferred to 4-mm gap electroporation cuvettes. 90 × 10⁶ cells in 600 µl are electroporated with 90 µg RNA (150 µg/ml) coding for the CSPG4-specific CAR per cuvette using a Gene Pulser Xcell at 500 V (square-wave pulse) for 5 ms.

### 1.3. SPION loading of CAR T-cells and crvoconservation

For SPION loading, the CAR T-cells (1× 10⁶ cells/ml) are incubated within 1 hour with the SPIONs (80 µg/ml) in OptiMem for 4h. Non-loaded cells serve as controls. After that, cells are washed and 8 aliquots of the SPION-loaded and non-loaded samples (1×10⁶ cells each) are taken for quality control purposes. The residual cells are cryoconserved in batches of 45 × 10⁶ cells (final conc. 25×10⁶ cells/ml).

### 1.4. Quality controls

To test if the cells are suitably loaded and functional, the quality controls performed:
1.4.1. Control of cell viability *in vitro:* non-loaded and SPION-loaded cells are stained with Annexin A5-Fitc/propidium iodide in Ringer's solution and are subsequently analyzed in flow cytometry. If the percentage of viable cells (Ax-PI-) in the SPION-loaded sample is ≥ 87%, the cell viability is appropriate.
1.4.2. Control of iron uptake *in vitro:* Cells are sedimented by centrifugation. After discarding the supernatant, the pellets are dried and lysed with 100 µl nitric acid at 95°C, 750 rpm for 15 min. 900 µL of water is added, followed by measurement of their iron concentration by atomic emission spectroscopy. If the SPION-loaded cells contain ≥ 0.9 pg Fe/cell, the SPION-loading is appropriate.
1.4.3. Control of tumor cell lysis capacity *in vitro:* Non-loaded and SPION-loaded CAR T-cells are incubated with tumor cells carrying the respective antigens for 3 days (ratio 1:20) in xCelligence system. If the cell index of tumor cells with SPION-loaded CAR T-cells is <50% of the cell index of untreated tumor cells (time point 72h), the tumor lysis capacity is appropriate.

### 2. Magnetic targeting of SPION-loaded CAR T-cells to solid tumor.

### 2.1. Bridging chemotherapy and lymphocyte depletion

Until infusion of CAR T-cells, a bridging chemotherapy might be necessary to control the tumor growth. 5 days before CAR T cell infusion lymphocytes are depleted with Cyclophosphamide 300 mg/m²/day (on day -5, -4, -3 i.v.).

### 2.2. Infusion of SPION-loaded CAR T-cells in the patient and magnetic enrichment.

Before the CAR T cell infusion, a control MRI (T2^{∗}) of the tumor region is performed as background control for T cell targeting. The tumor supplying vessels are imaged using contrast-based angiography and reconstructed in 3D. Based on the angiography, a suitable vessel is selected and the optimal path for magnetic movement evaluated.

Then, the respective number of cryoconserved CAR T-cells is thawed (1×10⁶ CAR+ T-cells/kg bodyweight, e.g. for a 100 kg patient: 100 ×10⁶ cells). Thawed cells are counted and viability is determined. CAR T-cells (1×10⁶ cells/kg bodyweight) are infused intra-arterially into the tumor supplying vascular system over a period of 60 minutes. During that time an electromagnet (1 Tesla) is placed downstream of the infusion position and moved slowly along the vessel. The magnet is placed within 60 minutes at various positions around the tumor.

### 2.3. Extravasation of CAR T-cells and tumor lysis

The magnetic force causes the accumulation of the CAR T-cells in the vessels of the tumor region. Subsequently, the CAR T-cells extravasate out of the vessel, and migrate into the tumor tissue. The SPION-loaded CAR T-cells bind to the tumor cells. The CAR T-cells are activated and express activation markers as well as GranzymeB and Fas ligand, which induce tumor cell death. SPION-loading of the CAR T-cells however suppresses cytokine release and tumor cell killing by pyroptosis, inhibiting the accompanying activation of myeloid cells and thereby preventing systemic inflammation. Mild local inflammation caused by damage-associated molecular patterns (DAMPs) released from killed tumor cells in contrast is beneficial to increase tumor immunogenicity and immune cell infiltration without overwhelming systemic immune activation.

### 2.4. Control of targeting efficacy and monitoring of side effects

Due to the SPION loading, the localization of CAR T-cells can be monitored. Before, 3 h and 5 days after the T cell infusion and magnetic targeting magnetic resonance imaging (MRI, T2^{∗}) is performed. MRI before the treatment serves as background control, 3h after serves as magnetic targeting control and 5 days after as T cell proliferation control, respectively.

Patients are observed for symptoms of cytokine release syndrome (headache, fever, chills, nausea, vomiting, diarrhea, dizziness, muscle/joint pain, fatigue, heart racing) or neuronal symptoms (headache, confusion/agitation, aphasia, seizures, tremor/twitching, balance problems), respectively.

Body temperature, breathing rate, blood pressure, heart rate, oxygen levels are measured regularly. Standard laboratory tests such as blood counts, electrolytes, indicators of kidney and liver function, CRP levels and ferritin levels are analyzed. (CRP: normal values: below 5 mg/l; worrisome >100 mg/l; Ferritin: normal; below 250 ng/ml; worrisome: > 3000 ng/ml)

In case of cytokine release syndrome, an anti-IL-6 receptor antibody such as tocilizumab and/or immune suppression (corticosteroids such as methylprednisolone or dexamethasone) may be given.

### Example 4 -Treatment of patients with solid tumors using SPION-loaded CAR-T-cells under magnetic fields.

### 1. MRI background control

Before the CAR-T cell infusion, a control MRI (T2^{∗}) of the tumor region is performed as background control for T cell targeting.

### 2. Preparation of the cells

The respective number of cryoconserved CAR-T-cells is thawed (1×10⁶ CAR-T-cells/kg bodyweight, e.g. for a 100 kg patient: 100 ×10⁶ cells). Thawed cells are counted and viability is determined. Cells are combined and placed into an infusion syringe, which is kept at 37°C in the incubator until use.

### 3. Imaging of tumor vascularization

The patient is placed onto the treatment table. The tumor and its supplying vessels are imaged using contrast-based angiography and reconstructed in 3D. Based on the angiography, a suitable vessel for intraarterial application of the SPION-loaded CAR-T-cells is selected. From that time on, the recorded tumor area of the patient should not be moved.

### 4. Simulation and test-run of application path (if available)

The optimal path for accumulation of nanoparticles is estimated based on 3D reconstruction of the tumor vessels. The optimal magnetic parameters are calculated. The distribution of the magnetized cells guided by the magnet is simulated. The robotic system is programmed to drive the path. A short test run is performed in the simulation tool.

### 5. Placement of catheter

Under local anesthesia a suitable 4F or 5F catheter is placed into a likewise suitable artery e.g. via an appropriate introducer. The location for gaining access to the vascular system of the patient to be treated has to be determined by the responsible team of physicians and radiologists but most probably it will be either the *arteria femoralis* or the *arteria radialis.* The tip of the catheter is moved to the tumor supporting vessel identified before by angiography and, if available, a suitable simulation tool. If necessary and possible, the part of the vessel downstream the branches that are supplying the tumor are blocked by inflating a balloon imbedded around the tip of the catheter. The CAR-T-cells are taken from the incubator and the infusion syringe is connected to the catheter via a t-valve connector. At the second inlet nozzle of the catheter a syringe with isotonic fluid is connected.

### 6. Infusion of cells

CAR-T-cells are administered via the catheter and magnetically accumulated with the magnetic field determined beforehand. The cells are slowly infused to the selected vessel of the patient over a time period of and flow rate that has to be adapted individually to the situation in each single patient (e.g. 15 minutes in pulses of 0.2 ml each minute). If necessary, the position of the magnetic field is shifted to reach an optimal distribution of the cells in the tumor. After the administration the first part of the administration procedure the catheter is flushed carefully with a suitable amount of isotonic fluid from the second syringe to ensure all CAR-T-cells being applied to the patient.

After the administration procedure is finished, the magnetic field will be kept in the tumor region for a suitable time, e.g. for 30 minutes. If it is possible and necessary, the magnetic field is moved in the tumor area by the robot and the respectable guidance software to ensure an optimal distribution of the CAR-T-cells in the tumor region.

### 7. Post-treatment

After the application and accumulation procedure of e.g. 45 minutes the electromagnet is switched off. The catheter and application equipment are removed and the wound is closed ensuring no residual bleeding.

### 8. Targeting control

4-6 h after magnetic targeting, the efficiency of CAR-T cell targeting to the tumor is monitored by control MRI with a suitable sequence (most probably T2- or T2^{∗}-weighted).

### Tables

Tables 1-3 provide the numbers of Figure 4C- 4E, respectively.

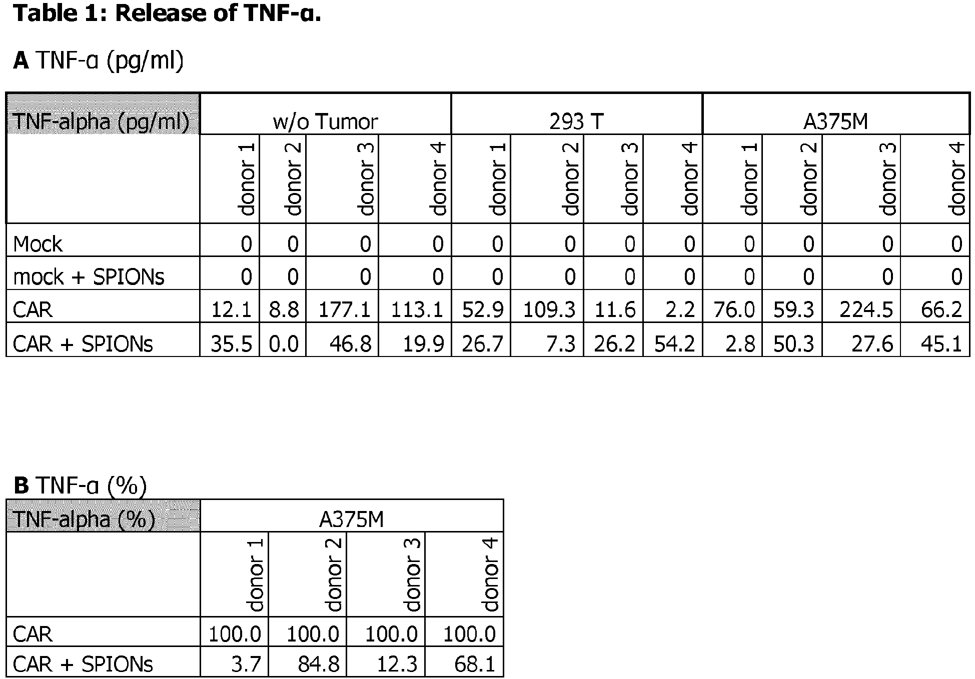

For the calculation of the percentages, the values of CAR w/o SPION were set to 100% and values of CAR with SPOIN was calculated in relation thereto.

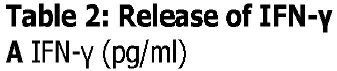

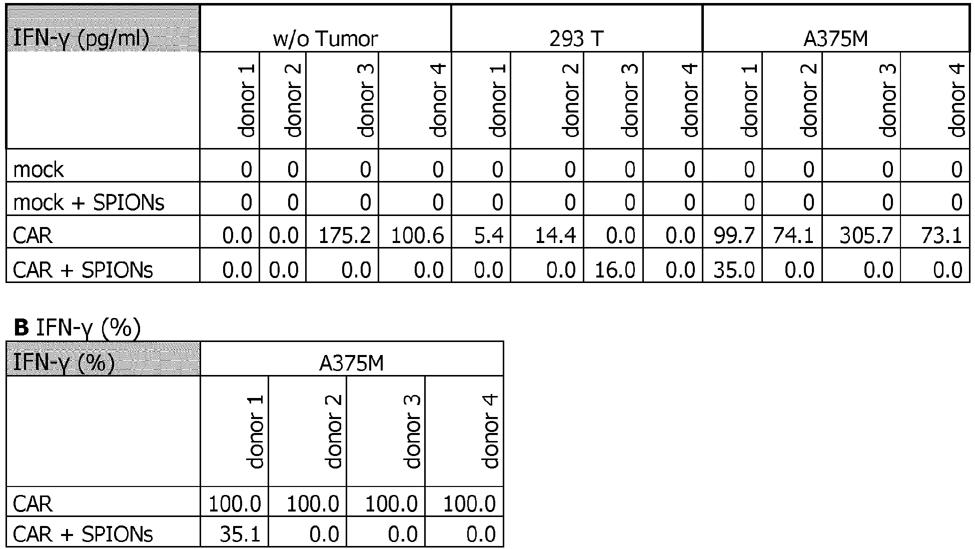

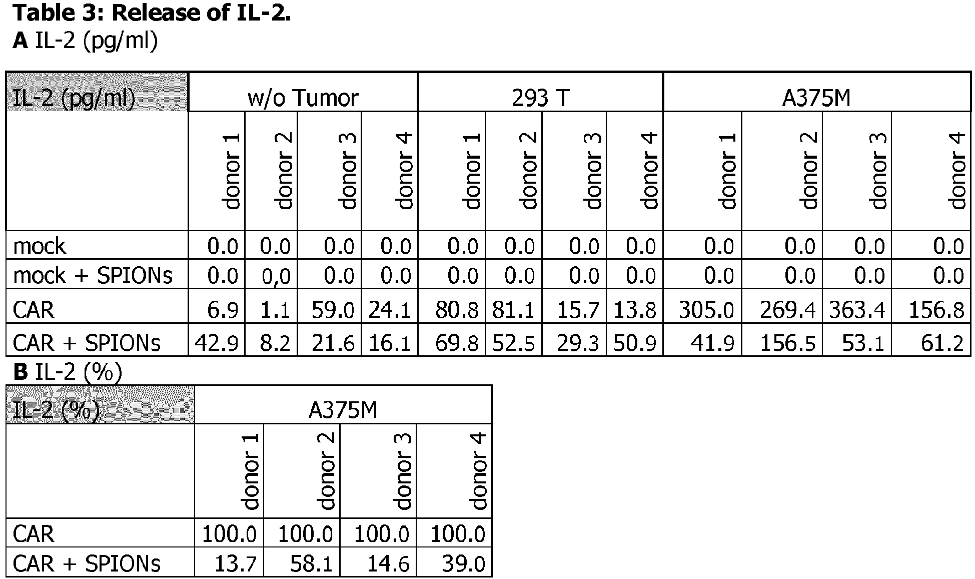

### Abbreviations

CCR2, CC chemokine receptor 2;
CCL2, CC chemokine ligand 2;
CCR5, CC chemokine receptor 5;
TLC, thin layer chromatography.

### References

1. Sadelain, M., I. Riviere, and R. Brentjens, Targeting tumours with genetically enhanced T lymphocytes. Nat Rev Cancer, 2003. 3(1): p. 35-45.
2. Sadelain, M., R. Brentjens, and I. Riviere, The basic principles of chimeric antigen receptor design. Cancer Discov, 2013. 3(4): p. 388-98.
3. Brudno, J.N. and J.N. Kochenderfer, Toxicities of chimeric antigen receptor T cells: recognition and management Blood, 2016. 127(26): p. 3321-30.
4. Curran, K.J., H.J. Pegram, and R.J. Brentjens, Chimeric antigen receptors for T cell immunotherapy: current understanding and future directions. J Gene Med, 2012. 14(6): p. 405-15.
5. Hou, A.J., L.C. Chen, and Y.Y. Chen, Navigating CAR-T cells through the solid-tumour microenvironment. Nat Rev Drug Discov, 2021. 20(7): p. 531-550.
6. Behm, F.G., et al., Human homologue of the rat chondroitin sulfate proteoglycan, NG2, detected by monoclonal antibody 7.1, identifies childhood acute lymphoblastic leukemias with t(4;11)(q21;q23) or t(11;19)(q23;p13) and MLL gene rearrangements. Blood, 1996. 87(3): p. 1134-9.
7. Chekenya, M., et al., The NG2 chondroitin sulfate proteoglycan: role in malignant progression of human brain tumours. Int J Dev Neurosci, 1999. 17(5-6): p. 421-35.
8. Godal, A., et al., Unexpected expression of the 250 kD melanoma-associated antigen in human sarcoma cells. Br J Cancer, 1986. 53(6): p. 839-41.
9. Harrer, D.C., et al., RNA-transfection of gamma/delta T cells with a chimeric antigen receptor or an alpha/beta T-cell receptor: a safer alternative to genetically engineered alpha/beta T cells for the immunotherapy of melanoma. BMC Cancer, 2017. 17(1): p. 551.
10. Shoshan, Y., et al., Expression of oligodendrocyte progenitor cell antigens by gliomas: implications for the histogenesis of brain tumors. Proc Natl Acad Sci U S A, 1999. 96(18): p. 10361-6.
11. Challier, J.C., et al., Characterization of first trimester human fetal placental vessels using immunocytochemical markers. Cell Mol Biol (Noisy-le-grand), 2001. 47 Online Pub: p. OL79-87.
12. Midwood, K.S. and D.M. Salter, Expression of NG2/human melanoma proteoglycan in human adult articular chondrocytes. Osteoarthritis Cartilage, 1998. 6(5): p. 297-305.
13. Petrini, S., et al., Human melanoma/NG2 chondroitin sulfate proteoglycan is expressed in the sarcolemma of postnatal human skeletal myofibers. Abnormal expression in merosin-negative and Duchenne muscular dystrophies. Mol Cell Neurosci, 2003. 23(2): p. 219-31.
14. Krug, C., et al., Stability and activity of MCSP-specific chimeric antigen receptors (CARs) depend on the scFv antigen-binding domain and the protein backbone. Cancer Immunol Immunother, 2015. 64(12): p. 1623-35.
15. Jin, H., et al., Magnetic Enrichment of Dendritic Cell Vaccine in Lymph Node with Fluorescent-Magnetic Nanoparticles Enhanced Cancer Immunotherapy. Theranostics, 2016. 6(11): p. 2000-2014.
16. Polyak, B., et al., High field gradient targeting of magnetic nanoparticle-loaded endothelial cells to the surfaces of steel stents. Proc Natl Acad Sci U S A, 2008. 105(2): p. 698-703.
17. Tukmachev, D., et al., An effective strategy of magnetic stem cell delivery for spinal cord injury therapy. Nanoscale, 2015. 7(9): p. 3954-8.
18. Boosz, P., et al., Citrate-Coated Superparamagnetic Iron Oxide Nanoparticles Enable a Stable Non-Spilling Loading of T Cells and Their Magnetic Accumulation. Cancers (Basel), 2021. 13(16).
19. Mühlberger, M., et al., Functionalization Of T Lymphocytes With Citrate-Coated Superparamagnetic Iron Oxide Nanoparticles For Magnetically Controlled Immune Therapy. Int J Nanomedicine, 2019. 14: p. 8421-8432.
20. Mühlberger, M., et al., Functionalization of T lymphocytes for magnetically controlled immune therapy: Selection of suitable superparamagnetic iron oxide nanoparticles. Journal of Magnetism and Magnetic Materials, 2019. 473: p. 61-67.
21. Mühlberger, M., et al., Loading of Primary Human T Lymphocytes with Citrate-Coated Superparamagnetic Iron Oxide Nanoparticles Does Not Impair Their Activation after Polyclonal Stimulation. Cells, 2020. 9(2).
22. Pai, A., et al., Dynamically Programmable Magnetic Fields for Controlled Movement of Cells Loaded with Iron Oxide Nanoparticles. ACS Applied Bio Materials, 2020. 3(7): p. 4139-4147.
23. Kiru, L., et al., In vivo imaging of nanoparticle-labeled CAR T cells. Proc Natl Acad Sci U S A, 2022. 119(6).
24. Sharpe, M.E., T-cell Immunotherapies and the Role of Nonclinical Assessment: The Balance between Efficacy and Pathology. Toxicol Pathol, 2018. 46(2): p. 131-146.
25. Berger, C., et al., Feasibility and limits of magnetically labeling primary cultured rat T cells with ferumoxides coupled with commonly used transfection agents. Mol Imaging, 2006. 5(2): p. 93-104.
26. Montet-Abou, K., et al., Transfection agent induced nanoparticle cell loading. Mol Imaging, 2005. 4(3): p. 165-71.
27. West, D.L., et al., Assessment and optimization of electroporation-assisted tumoral nanoparticle uptake in a nude mouse model of pancreatic ductal adenocarcinoma. Int J Nanomedicine, 2014. 9: p. 4169-76.
28. Friedrich, R.P., et al., Flow cytometry for intracellular SPION quantification: specificity and sensitivity in comparison with spectroscopic methods. Int J Nanomedicine, 2015. 10: p. 4185-201.
29. Waiczies, S., T. Niendorf, and G. Lombardi, Labeling of cell therapies: How can we get it right? Oncoimmunology, 2017. 6(10): p. e1345403.
30. Panet, E., et al., The interface of nanoparticles with proliferating mammalian cells. Nat Nanotechnol, 2017. 12(7): p. 598-600.
31. Rees, P., et al., The origin of heterogeneous nanoparticle uptake by cells. Nat Commun, 2019. 10(1): p. 2341.
32. Wilhelm, C. and F. Gazeau, Universal cell labelling with anionic magnetic nanoparticles. Biomaterials, 2008. 29(22): p. 3161-74.
33. McMahon, H.T. and E. Boucrot, Molecular mechanism and physiological functions of clathrin-mediated endocytosis. Nat Rev Mol Cell Biol, 2011. 12(8): p. 517-33.
34. Pombo Garcia, K., et al., Zwitterionic-coated "stealth" nanoparticles for biomedical applications: recent advances in countering biomolecular corona formation and uptake by the mononuclear phagocyte system. Small, 2014. 10(13): p. 2516-29.
35. Sanz-Ortega, L., et al., T cells loaded with magnetic nanoparticles are retained in peripheral lymph nodes by the application of a magnetic field. J Nanobiotechnology, 2019. 17(1): p. 14.
36. Starska, K., et al., The role of tumor cells in the modification of T lymphocytes activity--the expression of the early CD69+, CD71+ and the late CD25+, CD26+, HLA/DR+ activation markers on T CD4+ and CD8+ cells in squamous cell laryngeal carcinoma. Part I. Folia Histochem Cytobiol, 2011. 49(4): p. 579-92.
37. Dorrie, J., et al., BRAF and MEK Inhibitors Influence the Function of Reprogrammed T Cells: Consequences for Adoptive T-Cell Therapy. Int J Mol Sci, 2018.19(1).
38. Yan, L., et al., Fe2O3 nanoparticles suppress Kv1.3 channels via affecting the redox activity of Kvbeta2 subunit in Jurkat T cells. Nanotechnology, 2015. 26(50): p. 505103.
39. Yin, S., et al., Interactions of nanomaterials with ion channels and related mechanisms. Br J Pharmacol, 2019. 176(19): p. 3754-3774.
40. Elbialy, N.S., M.M. Fathy, and W.M. Khalil, Doxorubicin loaded magnetic gold nanoparticles for in vivo targeted drug delivery. Int J Pharm, 2015. 490(1-2): p. 190-9.
41. Munoz, L.E., et al., Colourful death: six-parameter classification of cell death by flow cytometry--dead cells tell tales. Autoimmunity, 2013. 46(5): p. 336-41.
42. Sauvola, J. and M. Pietikäinen, Adaptive document image binarization. Pattern Recognition, 2000. 33(2): p. 225-236.
43. Wiesinger, M., et al., Clinical-Scale Production of CAR-T Cells for the Treatment of Melanoma Patients by mRNA Transfection of a CSPG4-Specific CAR under Full GMP Compliance. Cancers (Basel), 2019. 11(8).

## Claims

1. A T-cell expressing a chimeric antigen receptor ('CAR T-cell') containing superparamagnetic iron-based particles ('loaded CAR T-cell') for use in treating a tumor, wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions.

2. The loaded CAR T-cell for use of claim 1, wherein the cytokine release comprises the release of TNFalpha, IFNgamma and/or IL-2, preferably wherein the cytokine release comprises at least two of TNFalpha, IFNgamma and/or IL-2, more preferably wherein the cytokine release comprises TNFalpha, IFNgamma and IL-2.

3. The loaded CAR T-cell for use of claims 1 or 2, wherein the reduced cytokine release is at least reduced by 20%, preferably at least 30%, more preferably at least 40%, and even more preferably at least 50%, compared to cytokine release by the unloaded CAR T-cell.

4. The loaded CAR T-cell for use of any of the preceding claims, wherein the loaded CAR T-cell is a 2^{nd} generation CAR T-cell, preferably wherein said 2^{nd} generation CAR T-cell comprises a co-stimulatory domain and an intracellular signaling domain, wherein the co-stimulatory domain comprises sections of CD28 or 4-1BB, more preferably wherein the co-stimulatory domain comprises section of CD28, and even more preferably wherein the co-stimulatory domain comprises section of CD28 and the intracellular signaling domain comprises section of CD3ζ, thereby forming a CD28-CD3ζ CAR.

5. The loaded CAR T-cell for use of any of the preceding claims, wherein the loaded CAR T-cell comprises from 0.5 to 4 pg iron per cell (Fe/cell), preferably from 0.55 to 3.5 pg Fe/cell, more preferably from 0.6 to 3.3 pg Fe/cell, more preferably from 0.65 to 3.1 pg Fe/cell, more preferably from 0.7 to 2.9 pg Fe/cell, more preferably from 0.8 to 2.7 pg Fe/cell, more preferably from 0.9 to 2.6 pg Fe/cell, more preferably from 1 to 2.5 pg Fe/cell, and most preferably around 1 pg Fe/cell.

6. The loaded CAR T-cell for use of any of the preceding claims, wherein the loaded CAR T-cell is a CSPG4-specific, a HER2-specific, a EGFRvIII-specific, a PSMA-specific, a CEA-specific, a MUC1-specific, a CD19-specific, a CD20-specific, a CD22-specific, a CD30-specific, a CD269-specific, a CD138-specific CAR T-cell, preferably wherein the loaded CAR T-cell is the CSPG4-specific CAR T-cell.

7. The loaded CAR T-cell for use of any of the preceding claims, wherein the loaded CAR T-cell is a theranostic agent, preferably wherein the theranostic agent is visible by MRI and/or ultrasound, more preferably wherein the MRI and/or ultrasound allows imaging controlled therapy.

8. The loaded CAR T-cell for use of any of the preceding claims, wherein the superparamagnetic iron-based particles have been cleaned of excess molecules by aqueous washing steps based on porous membrane filtration.

9. The loaded CAR T-cell for use of any of the preceding claims, wherein the superparamagnetic iron-based particles are selected from an iron oxide, iron (Fe), iron-cobalt, alnico, permalloy particles, preferably wherein the superparamagnetic iron-based particles are superparamagnetic iron oxide nanoparticles (SPI-ONs).

10. The loaded CAR T-cell for use of any of the preceding claims, wherein the superparamagnetic iron-based particles have been coated with citrate, oxalic acid, ascorbic acid, fatty acids and/or amino acids, preferably citrate.

11. The loaded CAR T-cell for use of any of the preceding claims, wherein the cell of the tumor is a cancer cell, preferably wherein the cancer cell is a solid tumor cell, more preferably wherein the solid tumor cell is a solid malignant tumor cell.

12. A CAR T-cell containing superparamagnetic iron-based particles (a 'loaded CAR T-cell'), wherein said loaded CAR T-cell exhibits a reduced cytokine release upon binding to a cell of the tumor expressing an antigen being recognized by the CAR of the CAR T-cell, compared to a CAR T-cell not containing superparamagnetic iron-based particles ('unloaded CAR T-cell') under the same conditions.

13. The CAR T-cell of claim 12, wherein the cytokine release is further defined in claims 2 or 3, wherein the loaded CAR T cell is further defined in any of claims 4-7, the superparamagnetic iron-based particles are further defined in any of claims 8-10, and/or wherein the cell of the tumor is further defined in claim 11.

14. An *in vitro* method of generating a CAR T-cell containing superparamagnetic iron-based particles (a 'loaded CAR T-cell') comprising, preferably consisting of, the steps
a. introducing a CAR mRNA into a T-cell; and
b. loading the T-cell of step (a) with superparamagnetic iron-based particles
whereby the loaded CAR T-cell is generated.

15. The method of claim 14, wherein the loaded CAR T cell is further defined in any of claims 4-7, and/or the superparamagnetic iron-based particles are further defined in any of claims 8-10.
